(19) Europäisches Patentamt
European Patent Office
Office européen des brevets

(11) Veröffentlichungsnummer: **0 162 383**
**A1**

# (12) EUROPÄISCHE PATENTANMELDUNG

(21) Anmeldenummer: 85105761.2

(22) Anmeldetag: 10.05.85

(51) Int. Cl.⁴: C 07 D 261/02
A 01 N 43/80, A 23 K 1/16
A 23 K 1/18, C 07 C 83/00
C 07 C 87/60, C 07 C 85/24

(30) Priorität: 17.05.84 DE 3418395

(43) Veröffentlichungstag der Anmeldung:
27.11.85 Patentblatt 85/48

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Ingendoh, Axel, Dr.
Unterste Dillenberg 18
D-5620 Velbert 15(DE)

(72) Erfinder: Berschauer, Friedrich, Dr.
Claudiusweg 9
D-5600 Wuppertal 1(DE)

(72) Erfinder: Becker, Benedikt, Dr.
Metzkausener Strasse 14
D-4020 Mettmann(DE)

(72) Erfinder: Stendel, Wilhelm, Dr.
In den Birken 55
D-5600 Wuppertal 1(DE)

(72) Erfinder: Homeyer, Bernhard, Dr.
Obere Strasse 28
D-5090 Leverkusen 3(DE)

(72) Erfinder: Scheer, Martin, Dr.
Herberts-Katernberg 7
D-5600 Wuppertal 1(DE)

(72) Erfinder: de Jong, Anno, Dr.
Stockmannsmühle 46
D-5600 Wuppertal 1(DE)

(72) Erfinder: Hänssler, Gerd, Dr.
Heymannstrasse 40
D-5090 Leverkusen 1(DE)

(72) Erfinder: Reinecke, Paul, Dr.
Steinstrasse 8
D-5090 Leverkusen 3(DE)

## (54) Biologisch aktive Mittel enthaltend substituierte Isoxazolidine sowie neue Isoxazolidine und ihre Herstellung.

(57) Die vorliegende Erfindung betrifft biologisch aktive Mittel die substituierte Isoxazolidine der Formel I

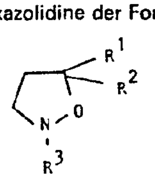

in welcher

$R^1$ für gegebenenfalls substituiertes Alkyl, Cycloalkyl, Aralkyl, Aryl, Heteroaryl oder Reste der Formel $-COR^4$ steht wobei

$R^4$ für gegebenenfalls substituiertes Alkyl, Aryl, Alkoxy, Alkylthio, Aroxy, Arylthio, Amino, Alkylamino, Dialkylamino, Arylamino, Arylalkylamino, Diarylamino, Cycloalkylamino, über Stickstoff gebundene gegebenenfalls substituierte heterocyclishche Reste steht,

$R^2$ für Wasserstoff sowie für die bei $R^1$ genannten Reste steht, wobei $R^1$ und $R^2$ gleich oder verschieden sein können,

$R^3$ Für gegebenenfalls substituiertes Alkyl, Cycloalkyl, Alkenyl, Alkinyl, Aryl sowie für gesättigte oder ungesättigte Heterocyclische Reste die gegebenenfalls substituiert sind, steht

./...

sowie deren Enantiomere, Diastereomere und Salze mit anorganischen und organischen Säuren als Wirkstoffe enthalten. Die Erfindung betrifft ferner neue substituierte Isoxazolidine sowie deren Enantiomere, Diastereomere und Salze mit anorganischen und organischen Säuren und Verfahren zu ihrer Herstellung.

BAYER AKTIENGESELLSCHAFT 5090 Leverkusen, Bayerwerk
Konzernverwaltung RP
Patentabteilung Rt/Kü-c

# Biologisch aktive Mittel enthaltend substituierte Isoxazolidine sowie neue Isoxazolidine und ihre Herstellung

Die vorliegende Erfindung betrifft biologisch aktive Mittel die substituierte Isoxazolidine als Wirkstoffe enthalten sowie neue Isoxazolidine und Verfahren zu ihrer Herstellung.

Substituierte Isoxazolidine sind bereits bekannt geworden. Es ist jedoch nichts über biologische Eigenschaften und insbesondere die Eignung dieser Isoxazolidine als Schädlingsbekämpfungsmittel und als Mittel zur Steigerung der Futterverwertung bei Tieren bekannt. (J. Org. Chem 44, 45 (1979) S. 835-839).

Es wurde gefunden, daß sich substituierte Isoxazolidine der Formel

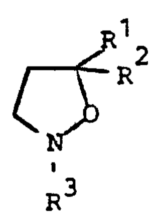

I

in welcher

$R^1$ für gegebenenfalls substituiertes Alkyl, Cycloalkyl, Aralkyl, Aryl, Heteroaryl oder Reste der Formel $-COR^4$ steht

wobei

$R^4$ für gegebenenfalls substituiertes Alkyl, Aryl, Alkoxy, Alkylthio, Aroxy, Arylthio, Amino, Alkylamino, Dialkylamino, Arylamino, Arylalkylamino, Diarylamino, Cycloalkylamino, über Stickstoff gebundene gegebenenfalls substituierte heterocyclische Reste steht,

$R^2$ für Wasserstoff sowie für die bei $R^1$ genannten Reste steht, wobei $R^1$ und $R^2$ gleich oder verschieden sein können,

$R^3$ für gegebenenfalls substituiertes Alkyl, Cycloalkyl, Alkenyl, Alkinyl, Aryl, Aralkyl, sowie für gesättigte oder ungesättigte heterocyclische Reste die gegebenenfalls substituiert sind, steht

sowie deren Enantiomere und Diastereomere und Salze mit anorganischen und organischen Säuren als biologisch aktive Mittel eignen. Biologisch aktive Mittel sind vor allem Schädlingsbekämpfungsmittel sowie Mittel zur Steigerung der Futterverwertung bei Tieren.

Bevorzugt seien genannt Verbindungen der Formel I in welcher

$R^1$ für $C_{1-5}$-Alkyl, $C_{3-6}$-Cycloalkyl, Phenyl-$C_{1-4}$-alkyl, Phenyl, Naphthyl, Pyridyl steht, die ein oder mehrfach durch Nitro, OH, CN, Halogen, $C_{1-4}$-Alkyl, $C_{3-6}$-Cycloalkyl, $C_{1-4}$-Alkoxy, $C_{1-4}$-Alkylthio, $C_{3-2}$-Alkylendioxy, $C_{1-4}$-Halogenalkyl, $C_{1-4}$-Alkoxycarbonyl, Carboxyl (COOH), Amino, $C_{1-4}$-Alkylamino, Di-$C_{1-4}$-alkylamino, Arylamino, Aryl-$C_{1-4}$-alkylamino, Aminocarbonyl-$C_{1-4}$-alkyl (-NHCOC$_{1-4}$-alkyl), $C_{1-4}$-Alkylsulfonylamino (-NHSO$_2$C$_{1-4}$-alkyl), $C_{1-4}$-Halogenalkoxy, $C_{1-4}$-Halogenalkylthio, $C_{1-4}$-Alkylendioxy, $C_{1-4}$-Halogenalkylendioxy, Aryl wie Phenyl, Aryloxy wie Phenoxy substituiert sein können, sowie für einen Rest der Formel $-COR^4$ steht, wobei $R^4$ für $C_{1-4}$-Alkyl, Phenyl, $C_{1-4}$-Alkoxy, $C_{1-4}$-Alkylthio, Phenoxy, Phenylthio, Amino, $C_{1-4}$-Alkylamino, Di-$C_{1-4}$-alkylamino, Phenylamino, Naphthylamino, Phenyl-$C_{1-4}$-alkylamino, Diphenylamino, $C_{3-6}$-Cycloalkylamino, Morpholino, Piperidino, Pyridino, Imidazolino, Piperazino, Triazolino steht, wobei diese Reste durch einen oder mehrere der oben aufgeführten Substituenten substituiert sein können,

$R^2$ für Wasserstoff steht oder für einen der unter $R^1$ angegebenen Reste steht, wobei $R_1$ und $R^2$ gleich oder verschieden sein können,

$R^3$ für $C_{1-10}$-Alkyl, $C_{3-8}$-Cycloalkyl, $C_{2-4}$-Alkenyl, $C_{2-4}$-Alkinyl, Phenyl, Naphthyl, Adamantyl steht, für $C_{1-6}$-Alkyl steht das durch Halogen, OH, CN, Amino, $C_{1-4}$-Alkylamino, $C_{1-4}$-Dialkylamino, $C_{1-4}$-Alkoxy, $C_{3-6}$-Cycloalkyl, gegebenenfalls substituiertes Phenyl, Naphthyl, Phenoxy, Phenylthio, Oxycar-

bonyl-$C_{1-4}$-alkyl ($OCO-C_{1-4}$-Alkyl), substituiert ist, für Phenyl das durch Halogen, $NO_2$, CN, OH, Amino, $C_{1-4}$-Alkylamino, Di-$C_{1-4}$-alkylamino, Carbonyl-($C_{1-4}$-alkoxy) ($-COOC_{1-4}$-Alkyl) substituiert ist, für gegebenenfalls substituiertes Phenoxy, Naphthoxy, Biphenyloxy steht.

Weiter seien besonders bevorzugt genannt Verbindungen der Formel I in welcher

$R^1$ für gegebenenfalls substituiertes Phenyl, Pyridyl, $C_{1-5}$-Alkyl, Naphthyl, sowie für einen Rest der Formel $-COR^4$ steht, wobei $R^4$ für $C_{1-4}$-Alkoxy insbesondere Methoxy oder Ethoxy steht, ferner steht $R^1$ für ein oder mehrfach gleich oder verschieden substituiertes Phenyl. Als Substituenten seien genannt Halogen wie Fluor, Chlor Brom, $C_{1-4}$-Alkyl, insbesondere Methyl, Propyl, Methoxy, Ethoxy, Nitro, Trifluormethyl, Trifluormethoxy, CN, $-COOCH_3$, COOH, Amino, $-NHCOCH_3$, Methylendioxy, Phenoxy das gegebenenfalls halogensubstituiert ist.

Ferner steht $R^1$ für $C_{1-5}$-Alkyl das ein oder mehrfach substituiert ist durch Halogen, Cyclopropyl, Cyclohexyl, Hydroxy, $C_{1-4}$-Alkoxy insbesondere Methoxy, Phenyl das gegebenenfalls durch Halogen, Methoxy substituiert ist.

Ferner steht $R^1$ für Pyridyl das durch Methyl oder Halogen insbesondere Chlor substituiert ist.

$R^2$ für Wasserstoff, Phenyl, $C_{1-4}$-Alkyl insbesondere Methyl, Propyl, Chlorphenyl, sowie für den Rest der Formel $-COR^4$ steht, wobei $R^4$ für $C_{1-4}$-Alkoxy, insbesondere Methoxy oder Ethoxy, Di-$C_{1-4}$-alkylamin insbesondere Diethylamin steht.

$R^3$ für $C_{1-6}$-Alkyl insbesondere Methyl, Ethyl, Propyl, t-Butyl, i-Pentyl, Pinakolyl, gegebenenfalls substituiert durch Cyclopropyl, Halogen, insbesondere Fluor, OH, Phenyl das gegebenenfalls durch Fluor, Trifluormethyl, substituiert ist, sowie für gegebenenfalls substituiertes Naphthyl, Adamantyl, Phenoxy, Halogenphenoxy, Naphthoxy, Biphenyloxy steht.

Im einzelnen seien Verbindungen der Formel I genannt denen die Reste die folgenden Bedeutungen besitzen:

| $R^1$ | $R^2$ | $R^3$ |
|---|---|---|
| $CH_3$ | $CO_2CH_3$ | 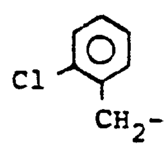 |
| 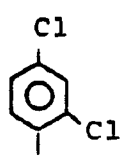 | H | 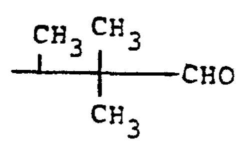 |
| $CONH_2$ | H | 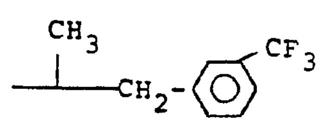 |
| $CO_2C_2H_5$ | H | 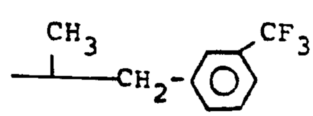 |

| | | |
|---|---|---|
| 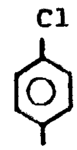 | H | 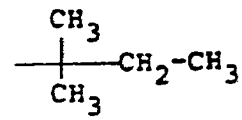 |
| 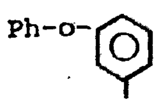 | H | 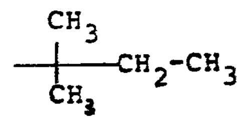 |
| 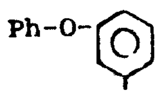 | H | 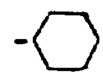 |
| 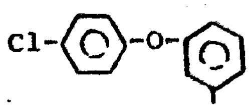 | H | 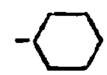 |
| HO-$CH_2$- | H | 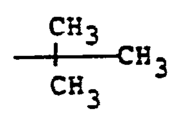 |
| 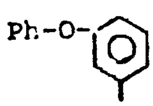 | H | 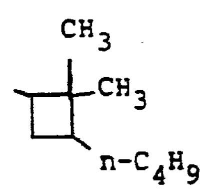 |
| 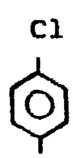 | H | 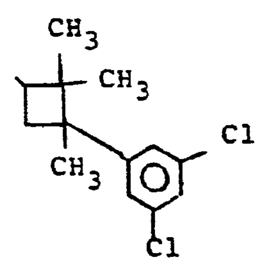 |

| | | |
|---|---|---|
| 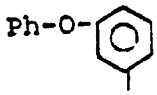 | H | 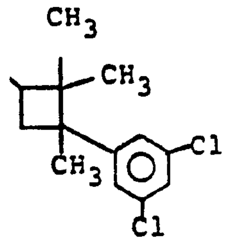 |
| 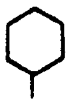 | $-C_2H_5$ | $-(CH_2)_5-CH_3$ |
| Ph | 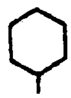 | 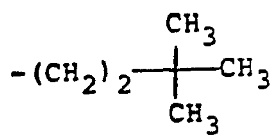 |
| $CO_2C_2H_5$ | $n-C_4H_9$ | 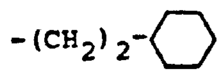 |
| $CONH_2$ | $n-C_3H_7$ | 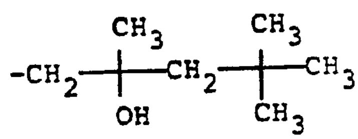 |
| 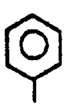 | 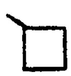 | 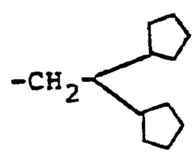 |
| 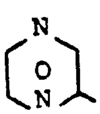 | $n-C_4H_9$ | 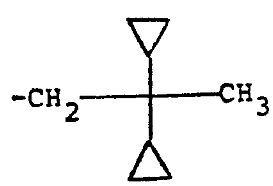 |

Die Bildung von Diastereomeren und Enantiomeren sei an folgendem Beispiel erläutert:

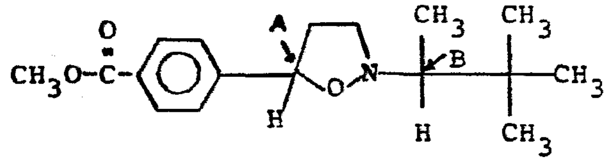

Die Kohlenstoffatome A und B sind chiral. Nach der Cahn-Ingold-Prelog Nomenklatur sind folgende Konfigurationen möglich:

1. A(R) + B(R)
2. A(S) + B(R)
3. A(R) + B(S)
4. A(S) + B(S)

Die Kombinationen 1. und 4. sind Enantiomere
Die Kombinationen 2. und 3. sind Enantiomere
Die Kombinationen 1. und 3. sind Diastereomere
Die Kombinationen 3. und 4. sind Diastereomere

Bevorzugt seien folgende Säuren genannt, die mit den Isoxazolidinen der Formel I Salze bilden können:

$HCl$, $H_2SO_4$, $HSO_4^-$, $H_3PO_4$, $HPO_4^-$, $HClO_4$, $HBr$, $HJ$, $HF$,

$HNO_3$, $H_2CO_3$, $HCO_3^-$, $H_3BO_3$, $HN_3$,

Essigsäure, Oxalsäure, Malonsäure, Bernsteinsäure,

Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure Trichloressigsäure, Fluoressigsäure, Phthalsäure, Naphthalinsulfonsäure, Nikotinsäure, Citronensäure, Ascorbinsäure.

Als Schädlingsbekämpfungsmittel seien bevorzugt Verbindungen der Formel I genannt in welcher

$R^3$ für gegebenenfalls substituiertes verzweigtes Alkyl steht; als Substituenten kommen dabei Halogen, insbesondere Chlor oder Fluor, Cyclopropyl, Cyclohexyl, Hydroxy, $C_{1-4}$-Alkoxy insbesondere Methoxy, Phenyl das gegebenenfalls ein- oder mehrfach durch Halogen, $C_{1-4}$-Halogenalkyl insbesondere $CF_3$ oder Methoxy substituiert ist infrage;

$R^1$ und $R^2$ die weiter oben genannten Bedeutungen und vorzugsweise Bedeutungen besitzen. Diese Schädlingsbekämpfungsmittel eignen sich besonders bevorzugt als Insektizide und Akarizide.

Als Futterzusatzmittel seien bevorzugt Verbindungen der Formel I genannt, in welcher

$R^1$ für gegebenenfalls substituiertes Aryl steht wobei die bei den vorzugsweise Definitionen genannten Reste auch hier besonders bevorzugt sind.

$R^2$ für Wasserstoff steht und

$R^3$ die oben genannten Bedeutungen und vorzugsweise Bedeutungen besitzt.

Es wurden ferner die neuen Verbindungen der Formel I

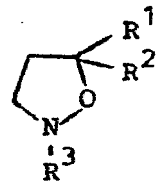

I

in welcher

$R^1$ und $R^2$ die oben angegebene Bedeutung sowie die oben angegebenen bevorzugten Bedeutungen besitzen und

$R^2$ für Alkyl mit mehr als 3 C-Atomen sowie für substituiertes Alkyl ferner für gegebenenfalls substituiertes Cycloalkyl, Alkenyl, Alkinyl, Aryl, sowie für gesättigte oder ungesättigte heterocyclische Reste die gegebenenfalls substituiert sind steht,

sowie ihre Enantiomeren und Diastereomeren gefunden.

Bevorzugt seien die neuen Verbindungen der Formel I genannt in der die Reste $R^1$ und $R^2$ die weiter oben angegebenen bevorzugten Bedeutungen besitzen und $R^3$ für $C_{3-10}$-Alkyl, für $C_{1-6}$-Alkyl das durch Halogen, OH,

CN, Amino, $C_{1-4}$-Alkylamino, Di-$C_{1-4}$-alkylamino, $C_{1-4}$-Alkoxy, $C_{3-6}$-Cycloalkyl, gegebenenfalls substituiertes Phenyl, Naphthyl, Phenoxy, Phenylthio, Oxycarbonyl-$C_{1-4}$-alkyl substituiert ist, für Phenyl das gegebenenfalls durch Halogen, $NO_2$, CN, OH, Amino, $C_{1-4}$-Alkylamino, Di-$C_{1-4}$-alkylamino, Carbonyl-$C_{1-4}$-alkoxy substituiert ist, steht.

Besonders bevorzugt seien die neuen Verbindungen der Formel I genannt, in der die Reste $R^1$ und $R^2$ die weiter oben als besonders bevorzugt aufgeführten Reste besitzen und

$R^3$ für i-Propyl, t-Butyl, i-Pentyl, Pinakolyl, sowie für Methyl, Ethyl, i-Propyl, t-Butyl, i-Pentyl, Pinakolyl steht die substituiert sind durch Cyclopropyl, Halogen insbesondere Fluor, OH, Phenyl das gegebenenfalls durch Fluor, Trifluormethyl substituiert ist sowie für Naphthyl steht.

Die neuen Verbindungen der Formel I

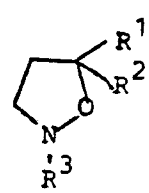

in welcher

$R^1$ und $R^2$ die oben angegebene Bedeutung sowie die oben angegebenen bevorzugten Bedeutungen besitzen und

$R^3$ für Alkyl mit mehr als 3 C-Atomen sowie für substituiertes Alkyl ferner für gegebenenfalls substituiertes Cycloalkyl, Alkenyl, Alkinyl, Aryl, sowie für gesättigte oder ungesättigte heterocyclische Reste die gegebenenfalls substituiert sind steht

werden erhalten, indem man Hydroxylamine der Formel II

$R^3$-NH-OH    II

in welcher

$R^3$ die oben angegebene Bedeutung hat,

mit Formaldehyd und Alkenen der Formel III

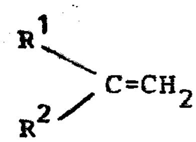

III

in welcher

$R^1$ und $R^2$ die oben angegebene Bedeutung haben,

umsetzt.

Hydroxylamine der Formel II sind teilweise neu. Es wurden die neuen Hydroxylamine der Formel V gefunden

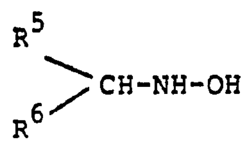

V

in welcher

$R^5$ für Verzweigtes Alkyl, Cycloalkyl, Alkenyl, Alkinyl, die gegebenenfalls durch Halogen, Aryloxy, Alkoxy, Halogenalkyl, Halogenalkoxy substituiert sind, ferner für durch Halogen, Alkoxy, Alkyl, Halogenalkyl, Halogenalkoxy substituiertes Aryl oder Aralkyl, steht

$R^6$ unabhängig von $R^5$ die dort angegebenen Bedeutungen hat,

sowie Salze mit anorganischen und organischen Säuren dieser Verbindungen.

Hydroxylamine der Formel V werden erhalten, indem man

a) Oxime der Formel VI

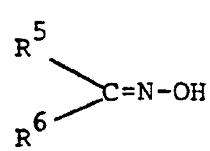

VI

in denen die Reste

$R^5$ und $R^6$ die oben angegebene Bedeutung haben

z.B. mit $NaBH_3CN$, Diboran in schwach saurer alkoholischer Lösung reduziert,

b) Nitroverbindungen der Formel

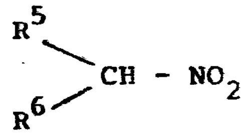

reduziert, z.B. mit Wasserstoff (Katalysator) oder Zinkstaub.

Oxime der Formel VI sind bekannt oder lassen sich analog zu bekannten Verfahren herstellen lassen.

Alkene der Formel III sind teilweise neu.

Es wurden die neuen Alkene der Formel VII gefunden

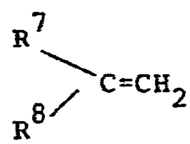

VII

in welcher

$R^7$ für Wasserstoff steht,

$R^8$ für Reste der Formeln steht,

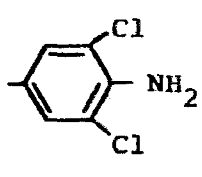
,
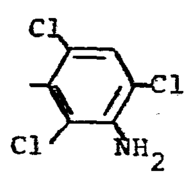
,
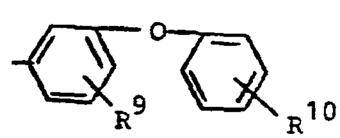

wobei

$R^9$ und $R^{10}$ unabhängig voneinander für Wasserstoff, Halogen (insbesondere Fluor) oder Trifluormethyl stehen, wobei $R^9$ und $R^{10}$ nicht gleichzeitig für Wasserstoff stehen dürfen.

Alkene der Formel VII werden erhalten indem man

a) Halogenalkyle der Formel VIII

$$R^8\text{-}CH_2\text{-}Hal \qquad VIII$$

in welcher

$R^8$ die oben angeebene Bedeutung hat

mit Triphenylphosphin und anschließend in Gegenwart einer Base mit Formaldehyd umsetzt, oder

b) einen Aldehyd der Formel IX

$$R^8\text{-}CHO \qquad IX$$

in welcher

$R^8$ die oben angegebene Bedeutung hat

in einer Wittig-Reaktion in an sich bekannter Weise olefiniert.

Das Verfahren zur Herstellung der neuen Verbindungen der Formel I kann dabei so durchgeführt werden, daß die Hydroxylamine der Formel II, Formaldehyd und die Alkene der Formel III zusammengegeben werden und zur Reaktion gebracht werden. Es läßt sich durch folgendes Formelschema darstellen:

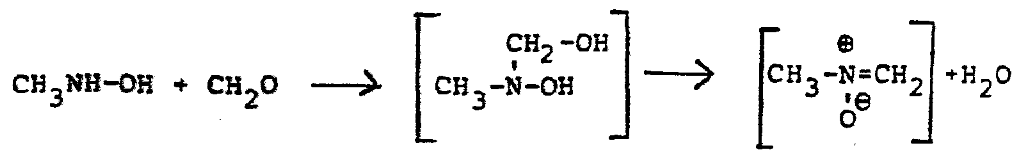

IV

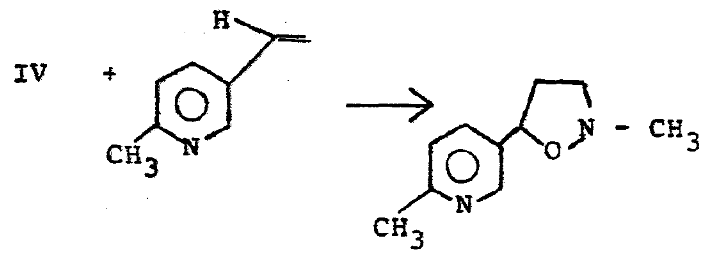

Besonders bevorzugt seien die folgenden bekannten Hydroxylamine der Formel II genannt:

$CH_3NHOH$, 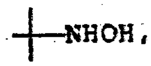, Phenyl-$CH_2$-NHOH, 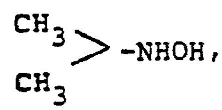,

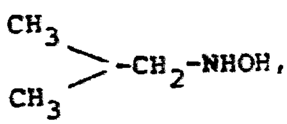, 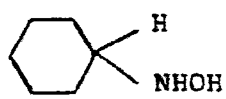, 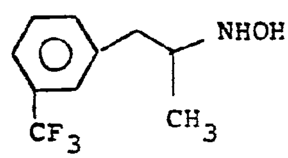

sowie 1-Adamantylhydroxylamin.

Besonders bevorzugt seien die folgenden neuen Hydroxylamine der Formel V genannt:

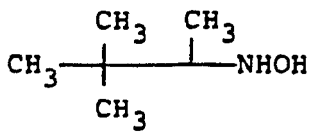 , 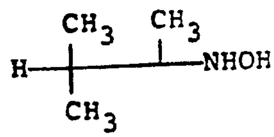 , 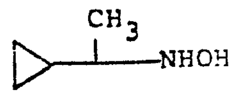

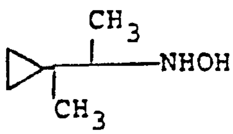 , 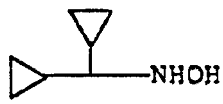 , 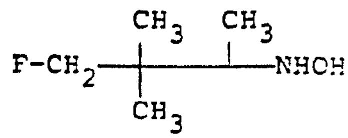 ,

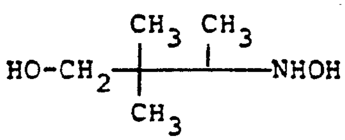 , 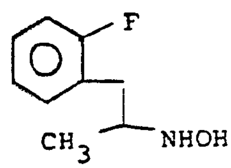

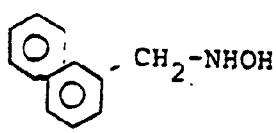 , 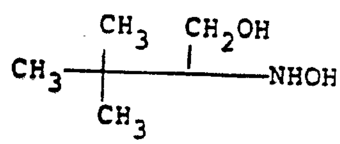 ,

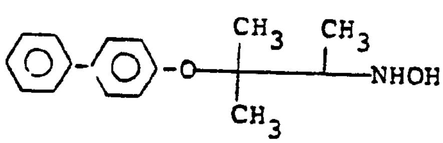 , 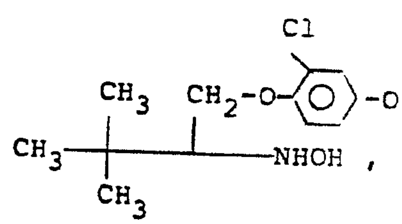 ,

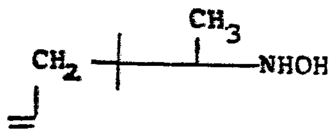 , 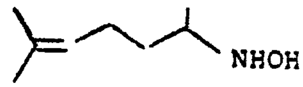 ,

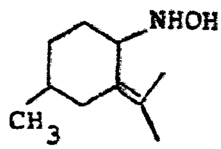

Die Reaktion wird bei Temperaturen von 0 - 100°C bevorzugt bei 100 - 140°C durchgeführt. Die Ausgangsverbindungen der Formeln II und III sowie Formaldehyd werden etwa in äquimolaren Mengen zusammengegeben. Formaldehyd kann auch in Überschuß (ca. 1-5 fach) eingesetzt werden.

Es wird normalerweise bei Normaldruck gearbeitet. Es ist auch möglich unter Druck zu arbeiten. Es wird bevorzugt in Gegenwart von Verdünnungsmitteln gearbeitet. Geeignete Verdünnungsmittel sind Kohlenwasserstoffe wie Toluol, Xylol, Benzol, Halogenkohlenwasserstoffe wie Chloroform, Alkohole wie Ethanol, Isopropanol, Ether wie tert.-Butyl-methylether.

Es ist auch möglich die Hydroxylamine der Formel II mit Formaldehyd zu versetz en und anschließend die Alkene der Formel III zum Reaktionsgemisch zuzugeben.

Es ist auch möglich nach einer besonders bevorzugten Verfahrensweise die Hydroxylamine der Formel II in Form

ihrer Salze mit anorganischen oder organischen Säuren, insbesondere HCl, sowie Formaldehyd, bevorzugt als Paraformaldehyd oder in Form seiner wäßrigen Formalinlösung (3-40 %) oder mit Trioxan, zur Reaktion zu bringen und anschließend das Alken der Formel III der Reaktionslösung zu geben.

Dabei werden die Salze der Hydroxylamine der Formel II in geeigneten Verdünnungsmitteln mit Formaldehyd wie z.B. Formalin versetzt und anschließend mit starken anorganischen oder organischen Basen neutralisiert.

Es wird bei Temperaturen von -20 bis +150°C und Normaldruck gearbeitet.

Die Ausgangsverbindungen werden etwa in äquimolarem Verhältnis zusammengegeben. Formaldehyd kann auch in Überschuß (ca. 1 - 5 fach) eingesetzt werden.

Als geeignete Verdünnungsmittel seien inerte organische Verdünnungsmittel wie Alkohole wie Methanol, Ethanol, Isopropanol, Kohlenwasserstoffe wie Toluol, DMF, DMSO, hochsiedende Ether Chlorkohlenwasserstoffe wie Chloroform, Dichlormethan, Ethylenchlorid genannt.

Als starke anorganische Basen seinen Alkali und Erdalkalihydroxide wie Natronlauge oder Kalilauge, Alkali und Erdalkalicarbonate und Hydrogencarbonate genannt.

Als geeignete organische Basen seien genannt: Trimethylamin, Triethylamin, N-Ethyldiisopropylamin, N,N-Dimethylanilin, N,N-Diethylanilin, N,N,N',N'-Tetramethylethylendiamin, N,N,N',N'-Tetraethylethylendiamin, Lutidin, Picolin, Pyridin.

Die Neutralisation erfolgt etwa bei -20 bis +160°C zu einem pH-Wert von 6-8.

Anschließend wird zum Reaktionsgemisch das Alken der Formel III sowie ein "Wasserschleppmittel" zugegeben.

Danach wird das Reaktionsgemisch je nach Siedepunkt des Wasserschleppmittels auf 110 bis 140°C erhitzt. Alkohol und Wasser werden mit Hilfe des Wasserschleppmittels azeotrop abdestilliert.

Als Wasserschleppmittel seien genannt:
Aromatische Kohlenwasserstoffe wie insbesondere Benzol, Toluol, Xylol, sowie halogenierte Kohlenwasserstoffe wie Chlorofom, Tetrachlorkohlenstoff.

Nachdem der Alkohol abdestilliert ist und das Wasser mit dem Wasserschleppmittel aus dem Reaktionsgemisch entfernt worden ist läßt man bei 110 - 140°C nachreagieren und arbeitet dann in üblicher Weise auf.

Wie bereits erwähnt sind die Hydroxylamine der Formel V neu. Das Verfahren zu ihrer Herstellung läßt sich durch folgendes Formelschema kennzeichnen:

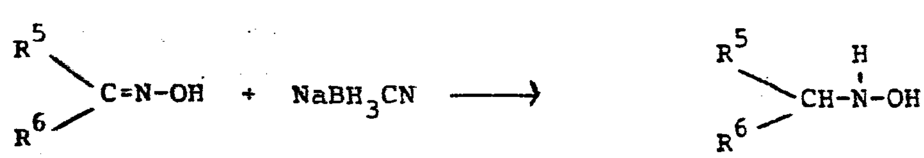

Die Reaktion wird bei -20 bis +100°C, bevorzugt bei 20°C, durchgeführt.

Es wird bei Normaldruck gearbeitet.

Die Ausgangskomponenten werden etwa in äquimolarem Verhältnis eingesetzt.

Es wird in Verdünnungsmitteln gearbeitet. Als solche seien genannt; Alkohole wie Methanol, Ethanol, Isopropanol, Ethylenglycol. Es kann auch in wäßrigen Alkoholen gearbeitet werden.

Es wird bei einem pH-Wert der Reaktionslösung von etwa 1-4 gearbeitet. Der pH-Wert kann durch eine geeichte Glaselektrode bestimmt werden. Ferner ist die Zugabe eines Farbindikators wie Bromkresolgrün oder Methylorange zur Einstellung des pH-Werts geeignet.

Die Aufarbeitung kann in an sich bekannter Weise durch Ansäuern des Ansatzes mit konzentrierter Säure wie Salzsäure zur Zersetzung des Cyanoborhydrids und anschließendem Alkalisieren, gefolgt von einer Extraktion des N-Alylhydroxylamins mit organischen Lösungsmitteln erfolgen (vgl. R. Borch J. Am. Chem. Soc. 93, 2897 (1971).

Besonders vorteilhaft ist es jedoch, nach Beendigung der Borhydridreduktion und Zersetzung des Borhydrids mit konzentrierter Säure wie Salzsäure den Ansatz im Wasserstrahlvakuum einzuengen, den Rückstand mit Chloroform oder Dichlormethan oder Alkoholen wie Ethanol, Isopropanol oder Methanol zu extrahieren. Die organische Lösung des N-Alkylhydroxylamin-Hydrochlorids wird mit Natriumsulfat oder anderen geeigneten Trocknungsmitteln getrocknet und am Rotationsverdampfer eingeengt.

Die Verbindungen der Formel V können auch in an sich bekannter Weise durch Reduktion der entsprechenden Nitroverbindungen mit Wasserstoff unter Katalyse von z.B. Palladium auf Kohle (vgl. US-P 3 173 953) oder durch Reduktion mit Zinkstaub in Eisessig, mit amalgamiertem Aluminium, mit Zinn-(II)-chlorid (vgl. Houben Weyl Methoden der org. Chemie Band 10/1 S. 1153) hergestellt werden.

Wie bereits erwähnt sind die Alkene der Formel VII neu. Das Verfahren zu ihrer Herstellung läßt sich durch folgendes Formelschema kennzeichnen:

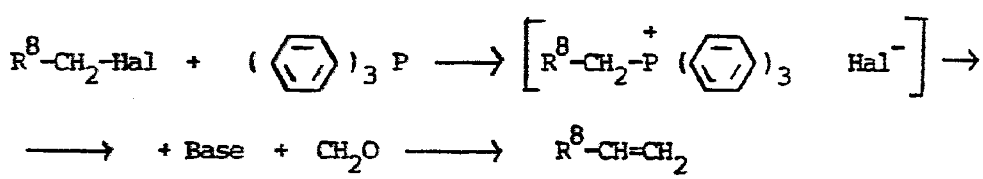


Als Halogenmethylverbindungen der Formel VIII seien genannt:

2-CN-, 2-$CH_3$-, 3-$NO_2$-, 3-$NO_2$-5-Methoxy-, 3-$NO_2$-5-Ethoxy-, 2,4-Dimethyl-benzylchlorid, Naphthylmethylchlorid.

Die Reaktion wird bei -20 bis +100°C, bevorzugt bei 20°C durchgeführt.

Es wird bei Normaldruck gearbeitet.

Die Ausgangsverbindungen werden in etwa äquivalenten Verhältnissen eingesetzt, wobei Formalin im 1-10fachen Überschuß eingesetzt wird.

Es wird in Gegenwart von Verdünnungsmitteln gearbeitet. Als Verdünnungsmittel seien genannt für die Olefinierung. Wasser, Alkohole (Methanol, Ethanol, Isopropanol).

Ein weiteres Verfahren zur Herstellung der Alkene der Formel VII läßt sich durch folgendes Formelschema kennzeichnen:

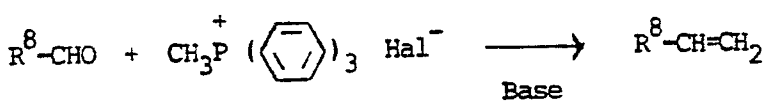


Als Aldehyde der Forml IX seien genannt: 2-Chlor-3,4-methylendioxy-, 2,4,6-Trichlor-5-amino-, 3,5-Dibrom-4-methoxy-, 3-Nitro-4-chlor-, 3-Phenoxy-, 3-(4-Chlorphenoxy)-, 3-(3-Trifluormethylphenoxy)-benzaldehyd, 3-Pyridylaldehyd.

Das Verfahren wird analog zu dem von R. Broos et al Synthetic Comm 6(1) 53-57 (1976), W.S. Emmerson Chem Rev. Band 45 (1949) S. 347 beschriebenen Verfahren durchgeführt.

Die Reaktion wird unter den für eine Wittig-Olefinierung üblichen Bedingungen durchgeführt. Die eingesetzten Aldehyde der Formel IX sind bekannt.

Als Olefinierungsmittel nach Wittig sind geeignete Phosphor Ylide wie $Ph_3P^{\oplus}-CH_3\ Cl^{\ominus}$ oder $Br^{\ominus}$ oder $J^{\ominus}$.

Die Reaktion wird bei 20 - 120°C, bevorzugt bei 80°C durchgeführt. Es wird bei Normaldruck gearbeitet.

Es wird in Verdünnungsmitteln gearbeitet. Als Verdünnungsmittel seien genannt:

Alkohole wie Methanol, Ethanol, Kohlenwasserstoffe wie Benzol, Toluol, Ether wie Diethylether, Dioxan, Tetrahydrofuran, Halogenkohlenwasserstoffe wie Dichlormethan.

Als Base wird verwendet: Amide wie Lithiumdiisopropylamid, $NaNH_2$, Hydride wie NaH, Alkali- und Erdalkalicarbonate wie $K_2CO_3$, Alkali- und Erdalkalihydroxide wie KOH, NaOH, Alkali- und Erdalkalialkoholate wie No-ter.-Butanolat.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii,

Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung

mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht der Tiere. Besonders wertvoll erweisen sich die Wirkstoffe bei der Aufzucht und Haltung von Jung- und Masttieren.

Als Tiere, bei denen die Wirkstoffe zur Förderung und Beschleunigung des Wachstums, zur Verbesserung der Futterverwertung sowie zur Verbesserung des Fleisch/Fett-Verhältnisses in Schlachtkörper eingesetzt werden können, seien beispielsweise folgende Nutz- und Ziertiere genannt:

Warmblüter wie Rinder, Schweine, Pferde, Schafe, Ziegen, Katzen, Hunde, Kaninchen, Pelztiere, z.B. Nerze, und Chinchilla, Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge des Wirkstoffs sowie die passende

Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen.

Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Die Wirkstoffe können als reine Stoffmischung oder in formulierter Form, also in Mischung mit nicht-toxischen inerten Trägerstoffen beliebiger Art, z.B. mit Trägerstoffen und in Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf

der Wirkstoff der Gesamtmenge oder nur Teilen des Futters und/oder des Trinkwassers zugegeben wird.

Die Wirkstoffe werden nach üblichen Methoden durch einfaches Mischen als reine Stoffmischung, vorzugsweise in fein verteilter Form oder in formulierter Form in Mischung mit eßbaren nicht-toxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise die Wirkstoffe in einer Gewichtskonzentration von etwa 0,01 bis 50, insbesondere 0,1 bis 10 ppm, enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die Wirkstoffe neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z.B. mit nicht-toxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auf zur Aufzucht und Mast anderer Tiere verwendet werden.

Mit den erfindungsgemäßen Wirkstoffen wurden mehrere Fütterungsversuche durchgeführt.

Dabei wurden folgende Ergebnisse erhalten:

Beispiel 1a

a) Tier-Charakteristik und Futter

| | |
|---|---|
| a 1) Ratten, weiblich | |
| a 2) Anzahl | 30 |
| a 3) Zucht | SPF Wistar, Züchtung Hagemann |
| a 4) Gewicht | 90-150 g |
| a 5) Zustand | gut |
| a 6) Futter | |

Rohnährstoffe *

| | |
|---|---|
| Rohprotein | 19,0 |
| Rohfett | 4,0 |
| Rohfaser | 6,0 |
| Asche | 7,0 |
| Wasser | 13,5 |
| N-freie Extraktst. | 50,5 |

Umsetzbare Energie:

| | |
|---|---|
| Kcal/kg | 3100 |
| KJ/kg | 13000 |

Mineralstoffe *

| | |
|---|---|
| Calcium | 0,9 |
| Phosphor | 0,7 |
| Magnesium | 0,2 |
| Natrium | 0,2 |

Vitamine **

Standard-Diät

| | |
|---|---|
| Vitamin A | 15000 IE |
| Vitamin $D_3$ | 600 IE |
| Vitamin E | 75 mg |
| Vitamin $K_3$ | 3 mg |
| Vitamin $B_1$ | 18 mg |
| Vitamin $B_2$ | 12 mg |
| Vitamin $B_6$ | 9 mg |
| Vitamin $B_{12}$ | 24 mcg |
| Nikotinsäure | 36 mg |
| Pantothensäure | 21 mg |
| Folsäure | 2 mg |
| Biotin | 60 mg |
| Cholin | 600 mg |
| Vitamin C | 36 mg |

Aminosäuren *

| | |
|---|---|
| Lysin | 0,9 |
| Methionin + Cystin | 0,6 |
| Phenylalanin+Tyrosin | 1,4 |
| Arginin | 1,1 |
| Histidin | 0,4 |
| Tryptophan | 0,2 |
| Threonin | 0,6 |
| Isoleucin | 0,9 |
| Leucin | 1,3 |
| Valin | 0,9 |

**Spurenelemente ****

| | |
|---|---|
| Magnan | 75,0 |
| Eisen | 135,0 |
| Kupfer | 13,0 |
| Zink | 70,0 |
| Jod | 0,9 |
| Fluor | 9,0 |

* % in der Diät (Mittelwert)
** mg in 1 kg Diät (Mittelwert)

b) **Behandlung der Tiere**

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 24)

b 2) 100 ppm (Wirkstoff Beispiel 49) (n = 6)

c) Ergebnis (Futteraufnahme, Wachstum, Futterverwertung) während der gesamten Versuchsperiode (13 Tage)

| | Futter-aufnahme (g) | Zuwachs (g) | Futter-verwer-tung (g/g) |
|---|---|---|---|
| c 1) Negativkontrolle | 177,3 | 35,4 | 5,01 |
| c 2) 100 ppm (Wirkstoff Beispiel 49) | 182,8 | 39,7 | 4,61 |

Beispiel 1b

a) Tier-Charakteristik und Futter

| | |
|---|---|
| a 1) Ratten, weiblich | |
| a 2) Anzahl | 27 |
| a 3) Zucht | SPF Wistar, Züchtung Hagemann |
| a 4) Gewicht | 90-150 g |
| a 5) Zustand | gut |
| a 6) Futter | |

Rohnährstoffe, Mineralstoffe, Vitamine, Ameisensäure und Spurenelemente und Behandlung der Tiere

- wie in Beispiel 1a -

Es wurden folgende Behandlungen geprüft:

b 1) Negativkontrolle (n = 21)
b 2) 25 ppm (Wirkstoff Beispiel 11) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 181,5 | 33,0 | 5,59 |
| c 2) 25 ppm (Wirkstoff Beispiel 11) | 183,3 | 36,8 | 4,99 |

Beispiel 1c

a) Tier-Charakteristik und Futter

| | |
|---|---|
| a 1) Ratten, weiblich | |
| a 2) Anzahl | 30 |
| a 3) Zucht | SPF Wistar, Züchtung Hagemann |
| a 4) Gewicht | 90-150 g |
| a 5) Zustand | gut |
| a 6) Futter | |

Rohnährstoffe, Mineralstoffe, Vitamine, Ameisensäure und Spurenelemente und Behandlung der Tiere

- wie in Beispiel 1a -

Es wurden folgende Behandlungen geprüft:

b 1) Negativkontrolle (n = 24)
b 2) 1 ppm (Wirkstoff Beispiel 6) (n = 6)
b 3) 25 ppm (Wirkstoff Beispiel 6) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 187,1 | 39,4 | 4,78 |
| c 2) 1 ppm (Wirkstoff Bsp.6) | 189,5 | 42,6 | 4,50 |
| c 3) 25 ppm (Wirkstoff Bsp. 6) | 193,5 | 46,0 | 4,20 |

Beispiel

Pyricularia-Test (Reis) systemisch

Lösungsmittel: 12,5 Gewichtsteile Aceton
Emulgator: 0,3 Gewichtsteile Alkylarylpolyglycolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele:

Beispiel

Pyricularia-Test (Reis) /protektiv

Lösungsmittel: 12,5 Gewichtsteile Aceton
Emulgator: 0,3 Gewichtsteile Alkylarylpolyglycolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung, bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele:

Beispiel

Laphygma-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:

Beispiel

Grenzkonzentrations-Test / Bodeninsekten

Testinsekt: Phorbia antiqua Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik:

## Herstellungsbeispiele

Allgemeine Arbeitsvorschrift:

Eine Lösung von 0,1 Mol des Alkens der Formel III in 250 ml Toluol wird auf 100°C erhitzt und mit einer alkoholisch wäßrigen Lösung, die erhalten wird indem man 0,11 Mol Hydroxylamin-Hydrochlorid der Formel II mit 0,11 Mol einer wäßrigen Formalin Lösung (33 %) versetzt, mit Kaliumhydroxidlösung (1 molar in Methanol) neutralisiert, vom Kaliumchlorid abfiltriert, versetzt.

Dabei destilliert zunächst Methanol ab, danach wird auf 140°C Außentemperatur erwärmt. Dabei destilliert Wasser azeotrop ab. Es wird weitere 5 Stunden unter Rückfluß gekocht. Man läßt erkalten, filtriert feste Rückstände ab und versetzt mit 100 ml 10 %iger Salzsäure. Man ethert das Gemisch zweimal mit 100 ml Ether aus, und alkalisiert den wäßrigen Rückstand mit konzentrierter wäßriger Natronlauge auf pH 8-10. Es wird erneut dreimal ausgeethert mit jeweils 100 ml Ether. Die vereinigten Etherauszüge werden über Natriumsulfat getrocknet, der Ether abdestilliert und der Rückstand im Hochvakuum destilliert oder mit ethanolischern Salzsäure zum Hydrochlorid umgesetzt oder mit ethanolischer Oxalsäure zum Oxalat umgesetzt.

Nach dieser allgemeinen Vorschrift werden die folgenden Verbindungen erhalten.

Allgemeine Formel:

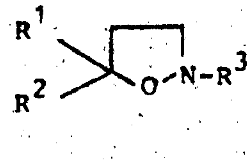

| $R^1$ | | $R^2$ | $R^3$ | Ausbeute | $^1H$-NMR-CCD($Cl_3$): $\delta$ /ppm | physik. Daten |
|---|---|---|---|---|---|---|
| 1 | 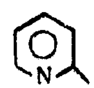 | H | $CH_3$ | 76 % | | Sdp. 65°C/ 0,1 mbar |
| 2 | 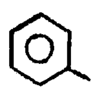 | 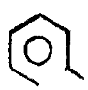 | $CH_3$ | 66 % | | Sdp. 46°C<br>Fp: 198 [HCl] |
| 3 | 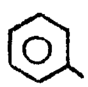 | $CH_3$ | $CH_3$ | 61 % | | Sdp.:65°C 0,25 mbar<br>Fp: 120°C [HCl] |
| 4 | 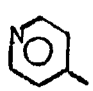 | H | $CH_3$ | 68,5 % | | Sdp: 140°C 0,22 mbar |
| 5 | 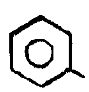 | H | 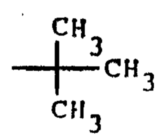 | 62,7 % | | Fp: 160°C [HCl] |

Fortsetzung:

| $R^1$ | | $R^2$ | $R^3$ | Ausbeute | $^1H$-NMR-CCD$(Cl_3)$: $\delta$/ppm | physik. Daten |
|---|---|---|---|---|---|---|
| 6 | 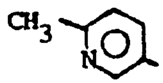 | H | $CH_3$ | 63,8 % | | Sdp: 90°C<br>2 mbar |
| 7 | 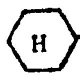 | $-(CH_2)_5-CO_2C_2H_5$ | $CH_3$ | 45 % | | Sdp: 138°C<br>0,02 mbar |
| 8 | 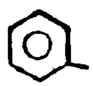 | $(CH_2)_2-CO_2C_2H_5$ | $CH_3$ | 57 % | | Sdp: 112-5°C<br>0,02 mbar |
| 9 | 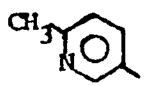 | H | 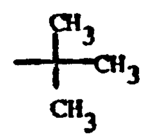 | 26 % | | Sdp: 82-5°C<br>0,2 mbar |
| 10 | 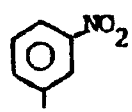 | H | $CH_3$ | 66 % | | Schmp: 148°C<br>/HCl/ |
| 11 | 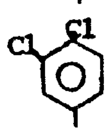 | H | $CH_3$ | 79 % | | Sdp: 98 %<br>0,2 mbar<br>Schmp: 107°C<br>/Oxalat/ |

Fortsetzung:

| | $R^1$ | $R^2$ | $R^3$ | Ausbeute | $^1H$-NMR-$CCD(Cl_3)$: $\delta$ /ppm | physik. Daten |
|---|---|---|---|---|---|---|
| 12 | 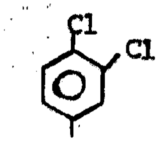 | H | 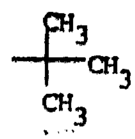 | 65 % | | Sdp: 115°C 0,3 mbar Schmp: 107°C /Oxalat7 |
| 13 | 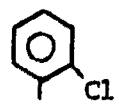 | H | $CH_3$ | 55 % | | Schmp: 93°C /Oxalat7 |
| 14 | 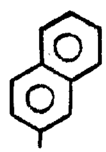 | H | $CH_3$ | 65 % | | Schmp: 169°C /Oxalat7 |
| 15 | 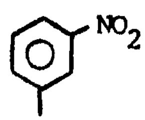 | H | 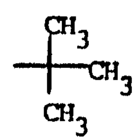 | 65 % | | Schmp: 139°C /Oxalat7 |
| 16 | 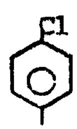 | H | $CH_3$ | 79 % | | Schmp: 130°C /Oxalat7 |

Fortsetzung:

| $R^1$ | | $R^2$ | $R^3$ | Ausbeute | $^1$H-NMR-CCD(Cl$_3$): $\delta$ /ppm | physik. Daten |
|---|---|---|---|---|---|---|
| 17 | 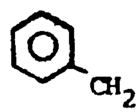 | $CO_2C_2H_5$ | $CH_3$ | 47 % | | Schmp: 139°C /Oxalat/ |
| 18 | 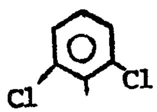 | H | $CH_3$ | 54 % | | Schmp: 156°C /Oxalat/ |
| 19 | 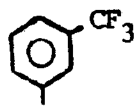 | H | $CH_3$ | 58 % | | Schmp: 130°C /Oxalat/ |
| 20 | 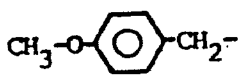 | $CO_2C_2H_5$ | $CH_3$ | 79 % | | Schmp: 129°C /Oxalat/ |
| 21 | 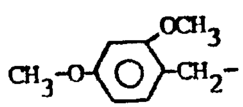 | $CO_2C_2H_5$ | $CH_3$ | 83 % | | Schmp: 131°C /Oxalat/ |

Fortsetzung:

| | $R^1$ | $R^2$ | $R^3$ | Ausbeute | $^1H$-NMR-CCD($Cl_3$): δ/ppm | physik. Daten |
|---|---|---|---|---|---|---|
| 22 | 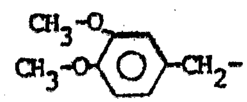 | $CO_2C_2H_5$ | $CH_3$ | 51 % | | Schmp: 142°C /Oxalat/ |
| 23 | 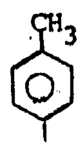 | H | $CH_3$ | 74 % | | Schmp: 148°C /Oxalat/ |
| 24 | 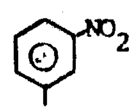 | H | 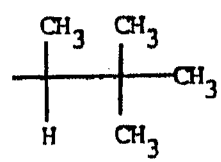 | 89 % | | Schmp: 120°C /Oxalat/ 142°C /HCl/ |
| 25 | 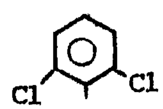 | H | 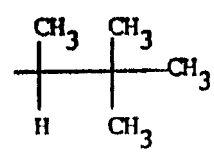 | 58 % | | Schmp: 139°C /Oxalat/ |
| 26 | 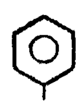 | H | $-CH_2$-Phenyl | 55 % | | Schmp: 124°C /Oxalat/ |

Fortsetzung:

| | $R^1$ | $R^2$ | $R^3$ | Ausbeute | $^1$H-NMR-CCD(Cl$_3$): δ /ppm | physik. Daten |
|---|---|---|---|---|---|---|
| 27 | 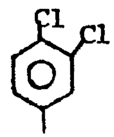 | $CH_3$ | 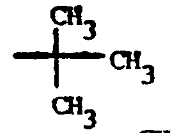 | 35 % | 1.06 /s7 | Öl |
| 28 | 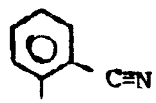 | H | 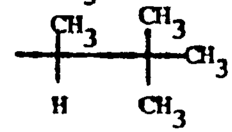 | 56 % | | Schmp: 146°C /Oxalat7 |
| 29 | 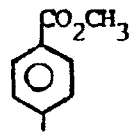 | H | $CH_3$ | 55 % | | Schmp: 158°C /Oxalat7 |
| 30 | 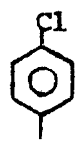 | $CH_3$ | 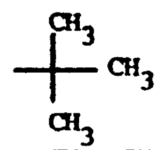 | 45 % | 1.53 /s7<br>1.48 /s7<br>1.2 /s7 | Öl |
| 31 | 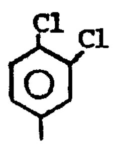 | $CH_3$ | 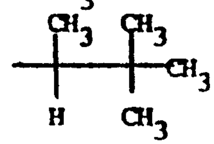 | 48 % | 1.51 /s7<br>1.48 /s7<br>0,96 /s7<br>0,95 /s7 | Öl |

Fortsetzung:

| $R^1$ | $R^2$ | $R^3$ | Ausbeute | $^1H$-NMR-CCD$(Cl_3)$: δ /ppm | physik. Daten |
|---|---|---|---|---|---|
| 32 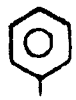 | 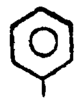 | 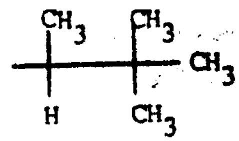 | 80 % | | Schmp: 133°C /Oxalat/ |
| 33 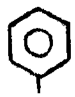 | 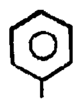 | $-CH_2$-Phenyl | 67 % | | Schmp: 143°C /Oxalat/ |
| 34 $CH_3$ | $CO_2CH_3$ | $-CH_2$-Phenyl | 15 % | | Schmp: 98 °C /Oxalat/ |
| 35 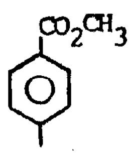 | H | $-CH_2$-Phenyl | 29 % | | Schmp: 99°C /Oxalat/ |
| 36 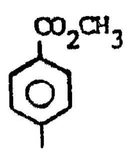 | H | 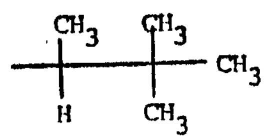 | 81 % | | Schmp: 162°C /HCl/ |

Fortsetzung:

| | $R^1$ | $R^2$ | $R^3$ | Ausbeute | $^1$H-NMR-CCD$(Cl_3)$: $\delta$ /ppm | physik. Daten |
|---|---|---|---|---|---|---|
| 37 | 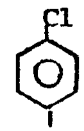 | $CH_3$ | 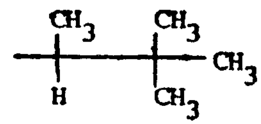 | 45 % | 1,53 /s7<br>1,46 /s7 | 0,97 /s7<br>Öl |
| 38 | 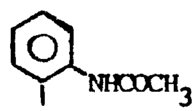 | $CH_3$ | 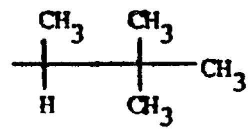 | 47,3 % | | Schmp: 118°C<br>/Oxalat7 |
| 39 | 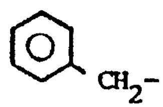 | $CO_2C_2H_5$ | 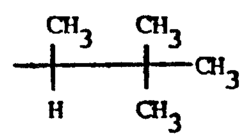 | 75 % | | Schmp: 122°C<br>/Oxalat7 |
| 40 | 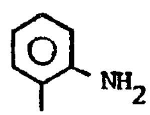 | $CH_3$ | 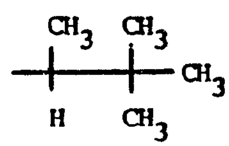 | 60 % | 0,91 /s7<br>0,95 /s7<br>1,63 /s7 | Öl |
| 41 | 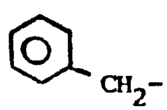 | $CO_2C_2H_5$ | $-CH_2$-Phenyl | 68 % | | Schmp: 117°C<br>/Oxalat7 |

Fortsetzung:

| $R^1$ | | $R^2$ | $R^3$ | Ausbeute | $^1$H-NMR-CCD(Cl$_3$): δ /ppm | physikalische Daten |
|---|---|---|---|---|---|---|
| 42 | 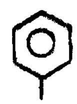 | H | $-CH_2-Ph$ | 67 % | | Schmp: 122°C /Oxalat/ |
| 43 | 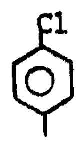 | H | 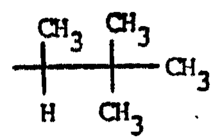 | 40 % | | Schmp: 98°C /HCl/ |
| 44 | 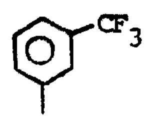 | H | 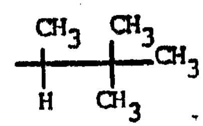 | 61 % | | Schmp: 156°C /HCl/ |
| 45 | 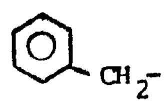 | $-CO_2C_2H_5$ | 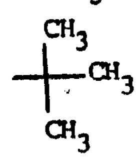 | 65 % | 1.10 /s/<br>3.08 /bs/ | Öl |
| 46 | $CH_3$ | $-CO_2CH_3$ | 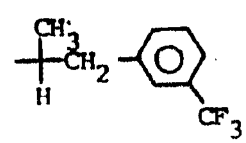 | 71 % | 0,81 /2x d/<br>1,29 /s/<br>1,30 /s/<br>3,78 /s/ | Öl |

Fortsetzung:

| | $R^1$ | $R^2$ | $R^3$ | Ausbeute | $^1H$-NMR-CCD($Cl_3$): δ /ppm | physikalische Daten |
|---|---|---|---|---|---|---|
| 47 | 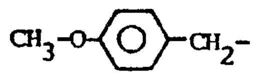 | $-CON(-C_2H_5)_2$ | $CH_3$ | 43 % | | Schmp: 95-100°C /Oxalat/ |
| 48 | 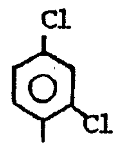 | H | 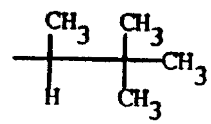 | 83 % | | Schmp: 158°C /HCl/ |
| 49 | 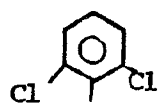 | H | 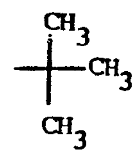 | 42 % | | Schmp: 135°C /Oxalat/ |
| 50 | 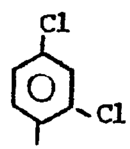 | H | $CH_3$ | 95 % | | Schmp: 132°C /Oxalat/ |
| 51 | 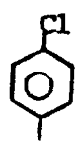 | H | 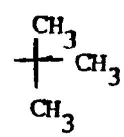 | 67 % | | Schmp: 100°C /Oxalat/ |

Fortsetzung:

| $R^1$ | | $R^2$ | $R^3$ | Ausbeute | $^1H$-NMR-$CCD(Cl_3)$: δ /ppm | physikal. Daten |
|---|---|---|---|---|---|---|
| 52 | 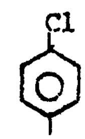 | H | 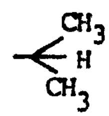 | 20 % | | Schmp: 130°C [Oxalat] |
| 53 | 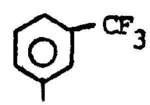 | H | 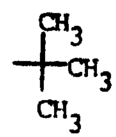 | 68,8 % | 1,23 [s]<br>5,03 [t] | Öl |
| 54 | 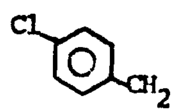 | $CO_2C_2H_5$ | $CH_3$ | 66 % | | Schmp: 152°C [Oxalat] |
| 55 | 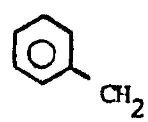 | $CON(C_2H_5)_2$ | $CH_3$ | 43 % | 2,60 [s]<br>2,90 [d x d] | Öl |
| 56 | 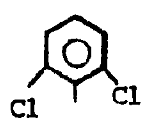 | H | 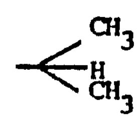 | 45 % | | Schmp: 169°C [HCl] |

Fortsetzung:

| | $R^1$ | $R^2$ | $R^3$ | Ausbeute | $^1H$-NMR-CCD($Cl_3$): $\delta$ /ppm | physikal. Daten |
|---|---|---|---|---|---|---|
| 57 | 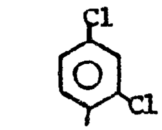 | $CO_2C_2H_5$ | 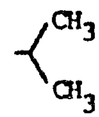 | 38 % | | Schmp: 122°C /Oxalat/ |
| 58 | 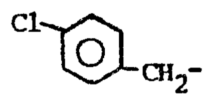 | $CO_2C_2H_5$ | 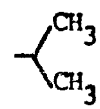 | 58 % | 3,01 /bs/ | Öl |
| 59 | 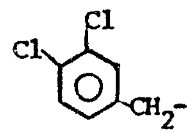 | $CO_2C_2H_5$ | 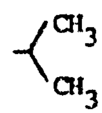 | 52 % | 3,03 /bs/ | Öl |
| 60 | 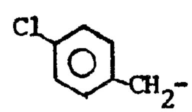 | $CO_2C_2H_5$ | 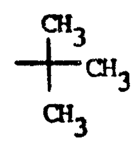 | 33 % | 1,11 /s/<br>3,06 /bs/ | Öl |
| 61 | 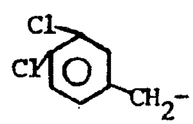 | $CO_2C_2H_5$ | 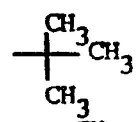 | 55 % | 1,08 /s/<br>3,05 /bs/ | Öl |
| 62 | 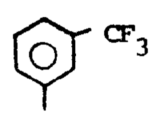 | H | 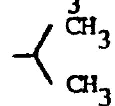 | 87 % | | Schmp. 123°C /Oxalat/ |

Fortsetzung:

| | $R^1$ | $R^2$ | $R^3$ | Ausbeute | $^1H$-NMR-CCD$(Cl_3)$: $\delta$ /ppm | physikal. Daten |
|---|---|---|---|---|---|---|
| 63 | 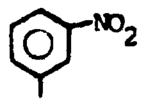 | H | 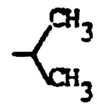 | 62 % | | Schmp: 142°C /Oxalat/ |
| 64 | 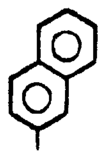 | H | 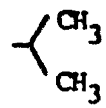 | 60 % | | Schmp: 125°C /Oxalat/ |
| 65 | 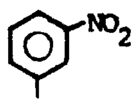 | H | 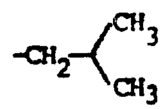 | 28,5 % | | Schmp: 106°C /Oxalat/ |
| 66 | 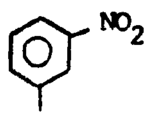 | H | 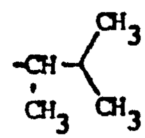 | 35 % | 5.05 /t/ | Öl |
| 67 | 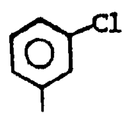 | H | $CH_3$ | 25 % | | Schmp: 97°C /Oxalat/ |

Fortsetzung:

| $R^1$ | | $R^2$ | $R^3$ | Ausbeute | $^1$H-NMR-CCD($Cl_3$): $\delta$ /ppm | physikal. Daten |
|---|---|---|---|---|---|---|
| 68 | 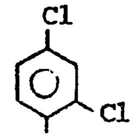 | H | 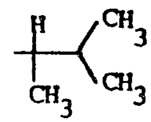 | 47,9 % | | Schmp: 141°C [Oxalat] |
| 69 | 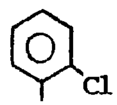 | H | 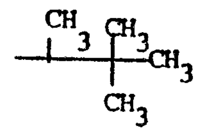 | 67 % | | Schmp: 159°C [HCl] |
| 70 | 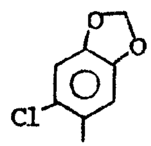 | H | $CH_3$ | 50,6 % | | Schmp: 134°C [Oxalat] |
| 71 | 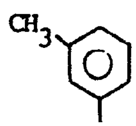 | H | 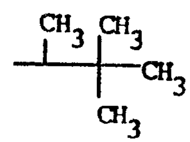 | 43 % | | Schmp: 100°C [Oxalat] |
| 72 | 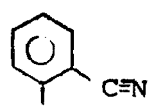 | H | 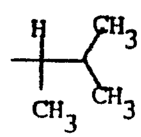 | 15 % | | Schmp: 95°C [Oxalat] |

Le A 22 983

Fortsetzung:

| | $R^1$ | $R^2$ | $R^3$ | Ausbeute | $^1$H-NMR-CCD($Cl_3$): $\delta$ /ppm | physikal. Daten |
|---|---|---|---|---|---|---|
| 73 | 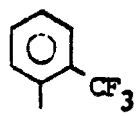 | H | 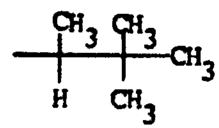 | 68 % | | Schmp: 160°C /HCl/ |
| 74 | 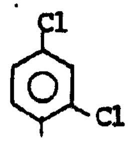 | H | 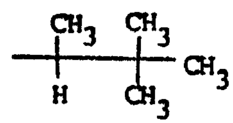 | 54 % | | Schmp: 110°C /HCl/ |
| 75 | 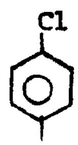 | H | $-CH_2-CF_3$ | 76,5 % | | Schmp: 123-5°C /HCl/ |
| 76 | 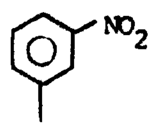 | H | 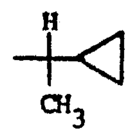 | 36 % | | Schmp: 114°C /Oxalat/ |
| 77 | 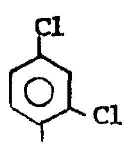 | H | 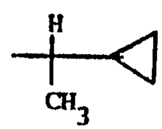 | 42,8 % | | Schmp: 132°C /Oxalat/ |

Fortsetzung:

| $R^1$ | | $R^2$ | $R^3$ | Ausbeute | $^1$H-NMR-CCD($Cl_3$): δ /ppm | physikal. Daten |
|---|---|---|---|---|---|---|
| 78 | 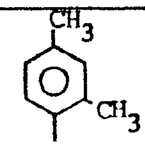 | H | 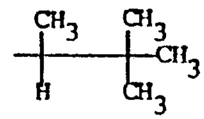 | 39 % | | Schmp: 176°C /HCl/ |
| 79 | 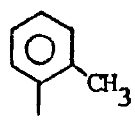 | H | 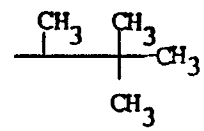 | 71,8 % | | Schmp: 181°C /HCl/ |
| 80 | $CH_3$ | $CO_2CH_3$ | 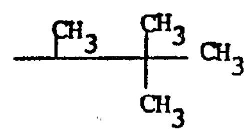 | 50 % | 0,66 /s/<br>1,35 /s/<br>1,36 /s/<br>3,63 /s/ | Öl |
| 81 | 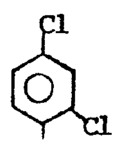 | H | 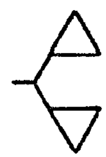 | 45 % | | Schmp: 125°C /Oxalat/ |
| 82 | 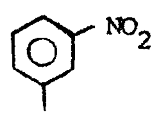 | H | 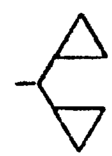 | 34 % | | Schmp: 104°C /Oxalat/ |

0162383

Fortsetzung:

| $R^1$ | | $R^2$ | $R^3$ | Ausbeute | $^1H$-NMR-$CCD(Cl_3)$: $\delta$/ppm | physik. Daten |
|---|---|---|---|---|---|---|
| 83 | 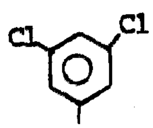 | $CH_3$ | 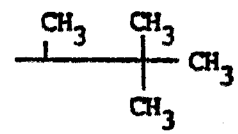 | 32 % | | Schmp: 131°C /Oxalat/ |
| 84 | 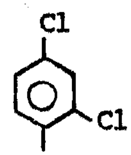 | H | 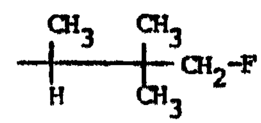 | 37 % | | Schmp: 150°C /HCl/ |
| 85 | 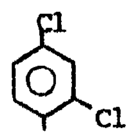 | H | 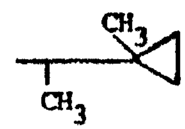 | 13 % | 0,33 /mult/<br>9,23 /t/ | Öl |
| 86 | 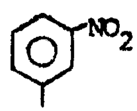 | H | 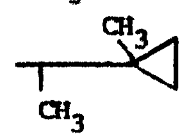 | 33 % | | Schmp: 150°C /HCl/ |
| 87 | 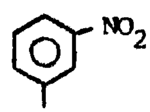 | H | 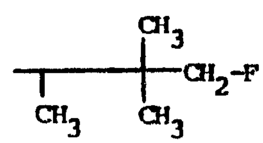 | 36 % | | Schmp: 132°C /Oxalat/ |

Fortsetzung:

| $R^1$ | | $R^2$ | $R^3$ | Ausbeute | $^1$H-NMR-CCD($Cl_3$): $\delta$ /ppm | physikalische Daten |
|---|---|---|---|---|---|---|
| 88 | 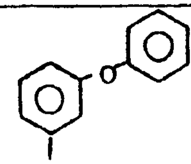 | H | 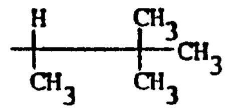 | 30 % | | Schmp: 91°C /HCl/ |
| 89 | 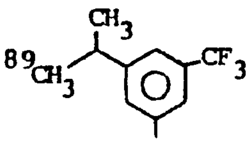 | $CH_3$ | 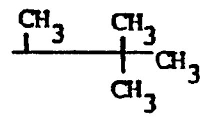 | 55 % | 0,96 /s/<br>1,00 /s/<br>1,50 /s/<br>1,56 /s/ | Öl |
| 90 | 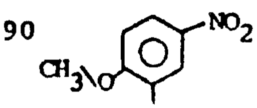 | H | 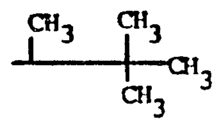 | 39 % | | Schmp: 185°C /HCl/ |
| 91 | 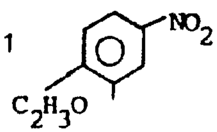 | H | 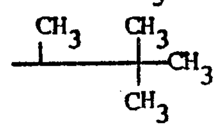 | 56 % | | Schmp: 185°C /HCl/ |
| 92 | 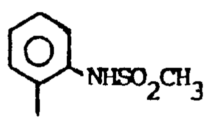 | $CH_3$ | 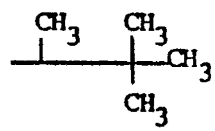 | 42 % | | Schmp: 205°C /HCl/ |
| 93 | 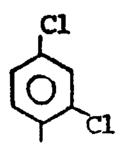 | H | 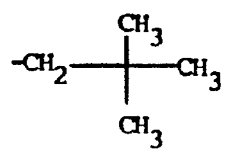 | 56 % | | Schmp: 91°C /Oxalat/ |

Fortsetzung:

| $R^1$ | | $R^2$ | $R^3$ | Ausbeute | $^1$H-NMR-CCD(Cl$_3$): δ /ppm | physikalische Daten |
|---|---|---|---|---|---|---|
| 94 | 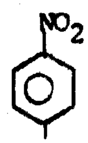 | H | $CH_3$ | 88 % | | Schmp: 175°C /HCl/ |
| 95 | 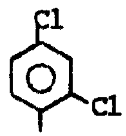 | H | 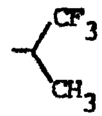 | 73,9 % | | Schmp: 123°C /HCl/ |
| 96 | 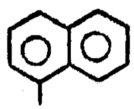 | H | 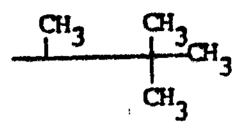 | 25,7 % | | Schmp: 184°C /HCl/ |
| 97 | 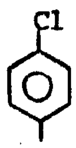 | H | 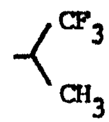 | 49 % | | Schmp: 92°C /HCl/ |
| 98 | 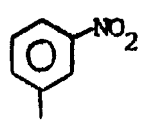 | H | 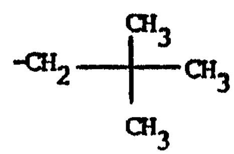 | 51 % | | Schmp: 133°C /Oxalat/ |

Fortsetzung:

| $R^1$ | | $R^2$ | $R^3$ | Ausbeute | $^1$H-NMR-CCD(Cl$_3$): $\delta$ /ppm | physik. Daten |
|---|---|---|---|---|---|---|
| 99 | 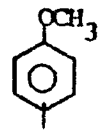 | H | $CH_3$ | 81 % | | Schmp: 142°C /Oxalat7 |
| 100 | 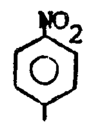 | H | 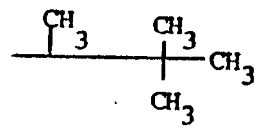 | 59 % | | Schmp: 103°C /Oxalat7 |
| 101 | 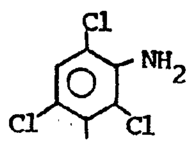 | H | 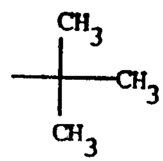 | 73 % | 1.18 /s7<br>5.53 /t7 | Öl |
| 102 | 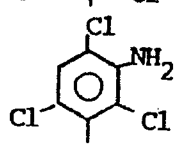 | H | 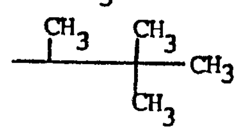 | 38 % | 0,93 /s7<br>5,56 /t7 | Öl |
| 103 | 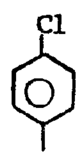 | H | 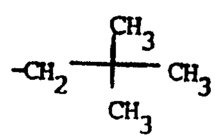 | 35 % | | Schmp: 99°C /Oxalat7 |

Fortsetzung:

| $R^1$ | | $R^2$ | $R^3$ | Ausbeute | $^1H$-NMR-CCD(Cl$_3$): δ /ppm | physikalische Daten |
|---|---|---|---|---|---|---|
| 104 | 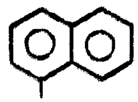 | H | $CH_3$ | 31 % | | Schmp: 165°C /HCl/ |
| 105 | 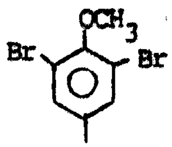 | H | 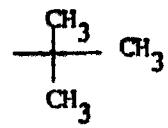 | 45,8 % | | Schmp: 188°C /HCl/ |
| 106 | 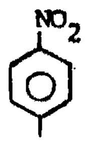 | H | 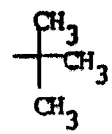 | 76 % | | Schmp: 113°C /Oxalat/ |
| 107 | 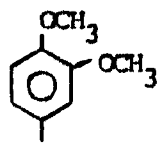 | H | 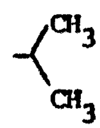 | 36,6 % | | Schmp: 135°C /Oxalat/ |
| 108 | 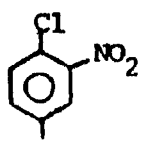 | H | 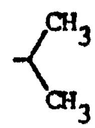 | 60,7 % | | Schmp: 109°C /Oxalat/ |

Fortsetzung:

| $R^1$ | | $R^2$ | $R^3$ | Ausbeute | $^1$H-NMR-CCD($Cl_3$): /ppm | physik. Daten |
|---|---|---|---|---|---|---|
| 109 | 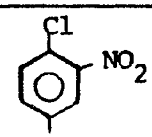 | H | $-CH_2-CF_3$ | 85 % | 5,13 /t/ | Öl |
| 110 | 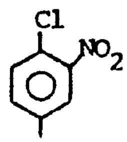 | H | 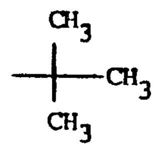 | 71 % | | Schmp: 117°C /Oxalat/ |
| 111 | 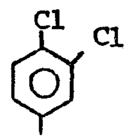 | 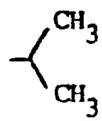 | 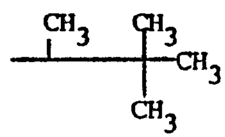 | 25 % | 0,80 /s/ | Öl |
| 112 | 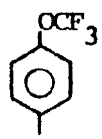 | H | 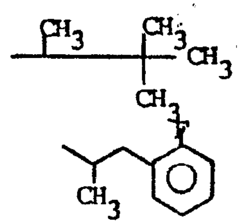 | 67,8 % | 0,81 /s/<br>4,13 /t/ | Öl |
| 113 | 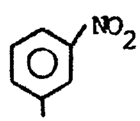 | H | | 45 % | 1,16 /d/<br>5,11 /t/ | Öl |

Fortsetzung:

| $R^1$ | | $R^2$ | $R^3$ | Ausbeute | $^1$H-NMR-CCD(Cl$_3$): [ppm] | physik. Daten |
|---|---|---|---|---|---|---|
| 114 | 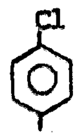 | 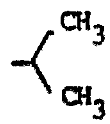 | 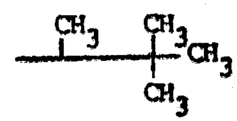 | 25 % | 1,16 [s] | Öl |
| 115 | $CH_3$ | $-CO_2CH_3$ | 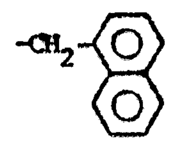 | 61 % | 1,16 [s]<br>1,18 [s]<br>3,73 [bs]<br>4,43 [d x d] | Öl |
| 116 | 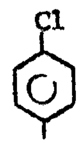 | 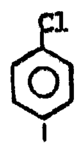 | 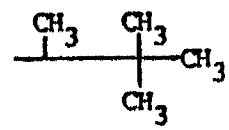 | 77 % | 1,01 [bs] | Öl |
| 117 | 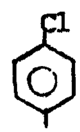 | 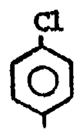 | 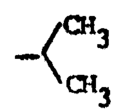 | 77 % | | Schmp: 162°C [Oxalat] |
| 118 | 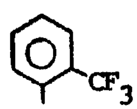 | H | 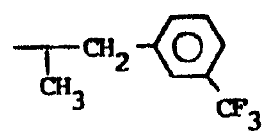 | 91 % | | Schmp: 127°C [HCl] |

Fortsetzung:

| $R^1$ | | $R^2$ | $R^3$ | Ausbeute | $^1H$-NMR-CCD($Cl_3$): /ppm | physik. Daten |
|---|---|---|---|---|---|---|
| 119 | 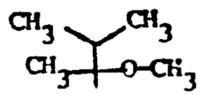 | H | 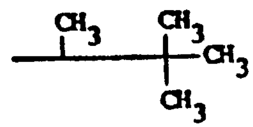 | 42 % | 3,26 [s]<br>1,43 [s]<br>1,30 [s]<br>1,00 [s]<br>0,88 [s] | Öl |
| 120 | 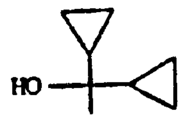 | H | 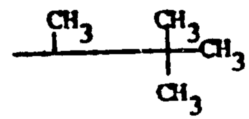 | 52 % | 0,40 [bs]<br>0,93 [s] | Öl |
| 121 | 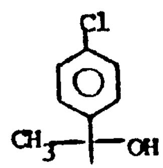 | H | 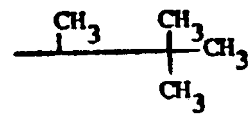 | 58,3 % | 1,00 [s]<br>1,60 [s]<br>1,58 [s] | Öl |
| 122 | 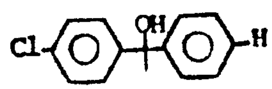 | H | 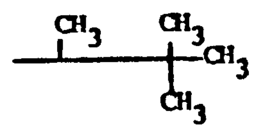 | 34 % | 5,06 [2x t]<br>0,83 [bs] | Öl |
| 123 | 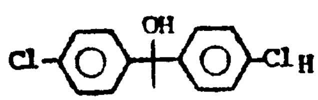 | H | 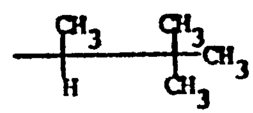 | 40 % | 4,96 [2xt]<br>0,76 [s] | Öl |
| 124 | 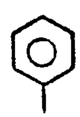 | H | 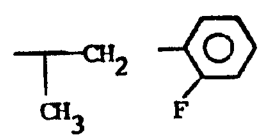 | 42 % | 5,06 [2xt]<br>1,06 [bd] | Öl |

Fortsetzung:

| | $R^1$ | $R^2$ | $R^3$ | Ausbeute | $^1H$-NMR-CCD($Cl_3$): /ppm | physik. Daten |
|---|---|---|---|---|---|---|
| 125 | 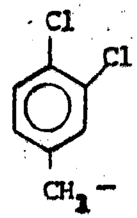 | $CO_2C_2H_5$ | $CH_3$ | 93 % | | 137°C /Oxalat/ |
| 126 | 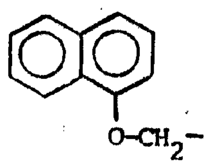 | H | 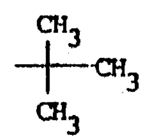 | 60 % | 2,05-2,50 /mult/<br>1,10 /s/ | |
| 127 | 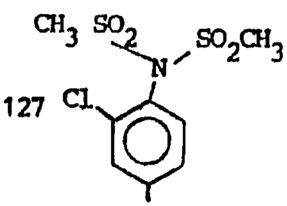 | H | 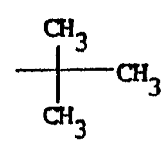 | 30 % | | 1,59-61°C |

Fortsetzung:

| | $R^1$ | $R^2$ | $R^3$ | Ausbeute | $^1H$-NMR-CCD(Cl$_3$): /ppm | physik. Daten |
|---|---|---|---|---|---|---|
| 128 | 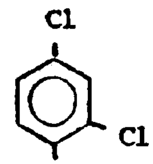 | H | 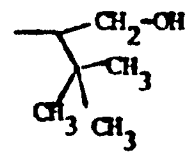 | 38 % | 6,00 /t/<br>1,16 /s/ | |
| 129 | 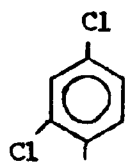 | H | 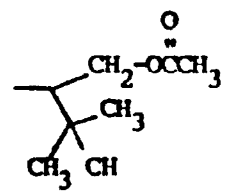 | 50 % | 2,01 /s/<br>1,01 /s/<br>0,98 /s/ | |
| 130 | 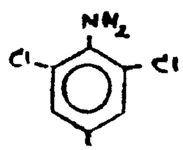 | H | 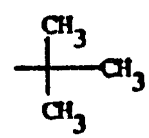 | 41 % | | 2 17°C /HCl/ |

Fortsetzung:

| $R^1$ | | $R^2$ | $R^3$ | Ausbeute | $^1H$-NMR-CCD$(Cl_3)$: /ppm | physik. Daten |
|---|---|---|---|---|---|---|
| 131 | 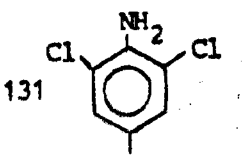 | H | 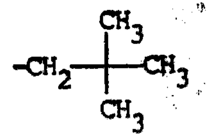 | 59 % | | 162°C /HCl/ |
| 132 | 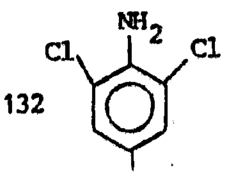 | H | $-CH_3$ | 73 % | 4,98 /t/<br>2,80 /s/ | |
| 133 | 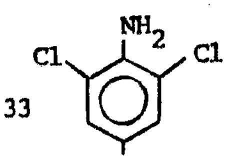 | H | 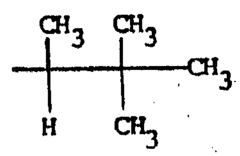 | 51 % | | 177°C /HCl/ |
| 134 | 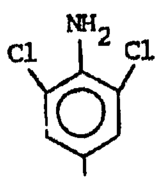 | H | 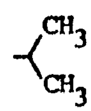 | 60 % | | 190°C /HCl/ |

Fortsetzung:

| | $R^1$ | $R^2$ | $R^3$ | Ausbeute | $^1$H-NMR-CCD(Cl$_3$): /ppm | physik. Daten |
|---|---|---|---|---|---|---|
| 135 | 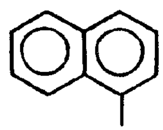 | H | 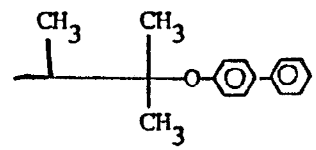 | 35 % | 5,75 /t/<br>1,45 /s/<br>1,35 /s/ | |
| 136 | 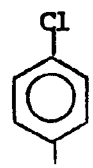 | H | 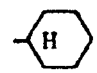 | 85 % | 5,05 /t/ | |
| 137 | 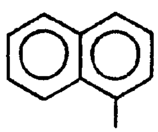 | H | 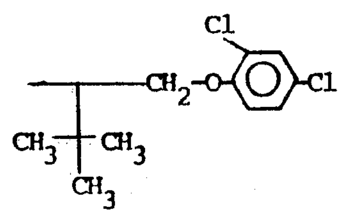 | | 5,68 /t/<br>1,16 /s/ | |
| 138 | 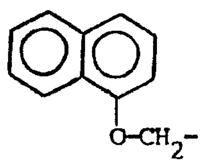 | H | 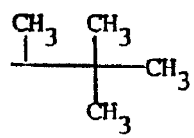 | 25 % | 0,98 /s/ | |

Fortsetzung:

| | $R^1$ | $R^2$ | $R^3$ | Ausbeute | $^1H$-NMR-CCD($Cl_3$): /ppm | physik. Daten |
|---|---|---|---|---|---|---|
| 139 | 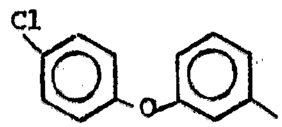 | H | 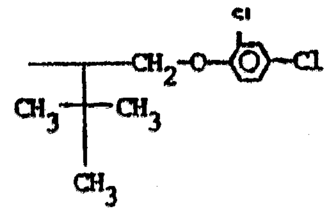 | 10 % | | 153°C /Oxalat/ |
| 140 | 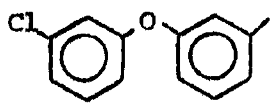 | H | 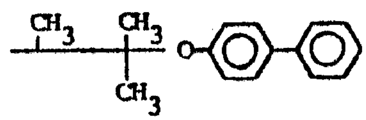 | 28 % | 4,96 /t/ | |
| 141 | 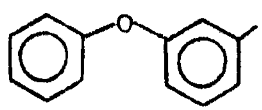 | H | 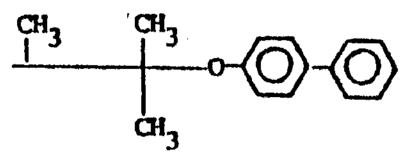 | 15 % | 5,00 /t/ | |

Fortsetzung:

| | $R^1$ | $R^2$ | $R^3$ | Ausbeute | $^1$H-NMR-CCD(Cl$_3$): /ppm | physik. Daten |
|---|---|---|---|---|---|---|
| 142 | 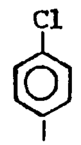 | H | 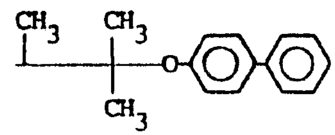 | 31 % | 5,02 /t/ | |
| 143 | 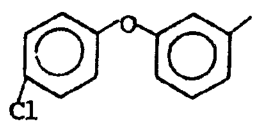 | H | 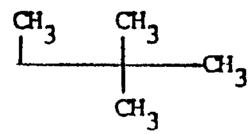 | 35 % | | 155°C /HCl/ |
| 144 | 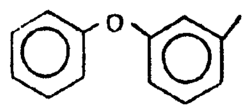 | H | 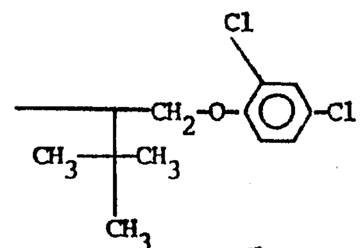 | 21 % | 5,01 /t/<br>1,00 /s/ | |
| 145 | $CH_3$- | $CO_2CH_3$ | 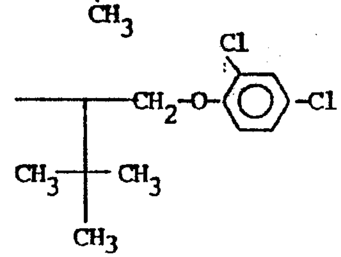 | 61 % | 3,76 /s/<br>3,64 /s/<br>1,56 /s/<br>1,40 /s/ | |

Fortsetzung:

| | $R^1$ | $R^2$ | $R^3$ | Ausbeute | $^1H$-NMR-CCD($Cl_3$): /ppm | physik. Daten |
|---|---|---|---|---|---|---|
| 146 | 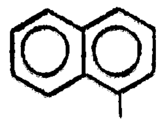 | H | 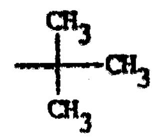 | 65 % | 5,60 /t/<br>1,23 /s/ | |
| 147 | 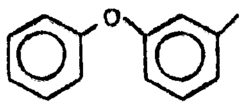 | H | 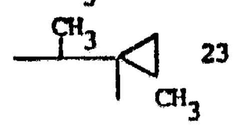 | 23 % | 4,83 /t/<br>1,30 /s/<br>1,13 /s/<br>1,00 /s/ | |
| 148 | 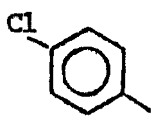 | H | 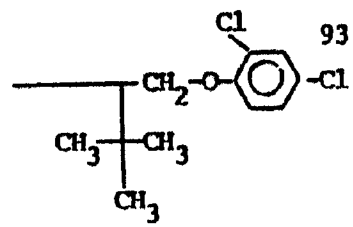 | 93 % | | 172°C /HCl/ |
| 149 | 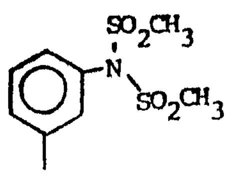 | H | 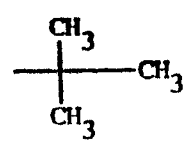 | 25 % | | 146°C |

Fortsetzung:

| | $R^1$ | $R^2$ | $R^3$ | Ausbeute | $^1$H-NMR-CCD(Cl$_3$): /ppm | physik. Daten |
|---|---|---|---|---|---|---|
| 150 | 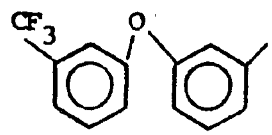 | H | 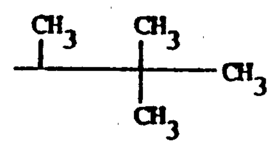 | 50,5 % | 4,83 /7<br>0,8 /s/ | |
| 151 | 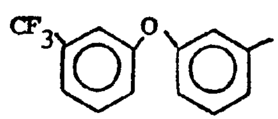 | H | 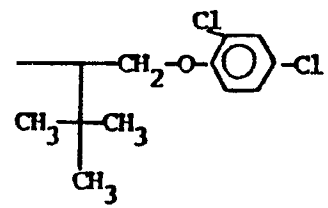 | 78 % | 5,20 /t/<br>1,20 /s/ | |
| 152 | 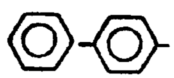 | H | 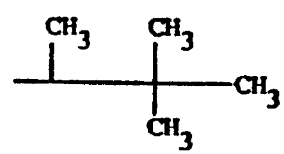 | 61 % | | 42°C |
| 153 a) | 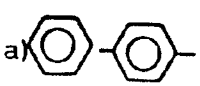 | H | 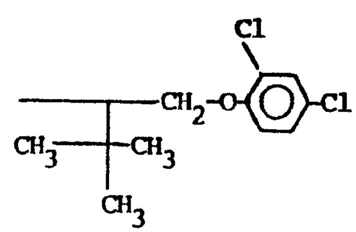 | 53 % | 5,05 /t/<br>1,08 /s/ | |

Fortsetzung:

| | $R^1$ | $R^2$ | $R^3$ | Ausbeute | $^1H$-NMR-$CDCl_3$: /ppm | physik. Daten |
|---|---|---|---|---|---|---|
| 153 b) | 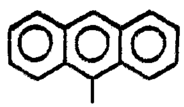 | H | 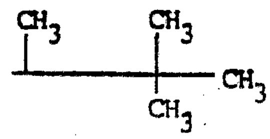 | 56 % | 6,0-6,5 /m/<br>1,00 /s/ | |
| 154 | 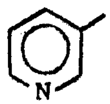 | H | $CH_3$ | 18 % | | 95°C (Oxalat) |
| 155 | 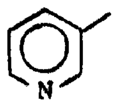 | H | 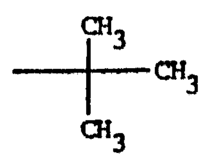 | 39 % | | 135°C (Oxalat) |
| 156 | 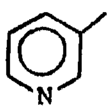 | H | 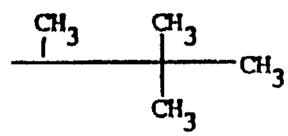 | 43 % | | 149°C (Oxalat) |
| 157 | 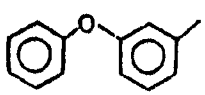 | H | 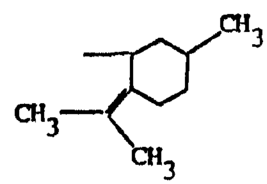 | 27 % | 5,01 /t/<br>0,60-1,00 /m/ | |

Fortsetzung:

| | $R^1$ | $R^2$ | $R^3$ | Ausbeute | $^1H$-NMR-CCD(Cl$_3$): /ppm | physik. Daten |
|---|---|---|---|---|---|---|
| 158 | 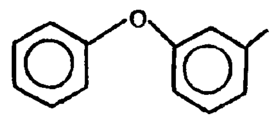 | H | 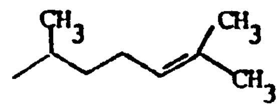 | 20,5 % | 4,90 /t/ 1,0-1,5 /m/ | |
| 159 | 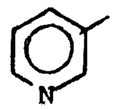 | H | 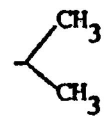 | 32 % | 5,00 /t/ 1,17 /d/ | |
| 160 | 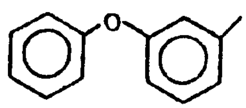 | H | 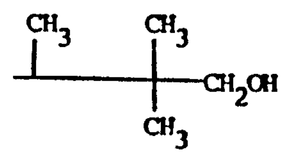 | 15 % | 4,85 /m/ | |
| 161 | 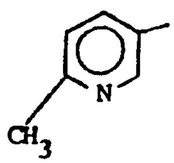 | H | 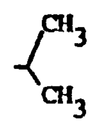 | 65 % | | Kp: 90-93°C 0,1 mbar |

Fortsetzung:

| | $R^1$ | $R^2$ | $R^3$ | Ausbeute | $^1H$-NMR-CCD$(Cl_3)$: /ppm | physik. Daten |
|---|---|---|---|---|---|---|
| 162 | 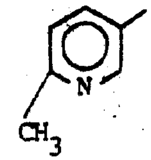 | H | 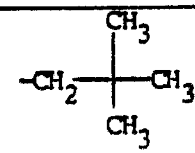 | 26 % | | Kp: 95-103°C 0,2 mbar |
| 163 | 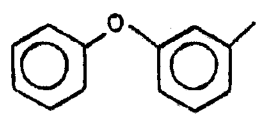 | H | 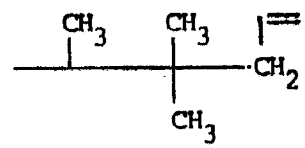 | 25 % | 4,95 /t/<br>0,80-1,1 /m/ | |
| 164 | 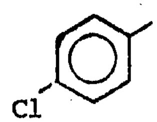 | H | 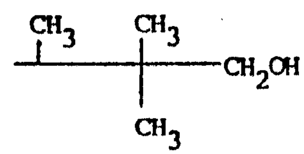 | 41 % | 4,93 (t)<br>3,80 (bs) | Öl |
| 165 | 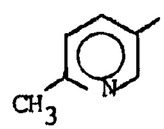 | H | 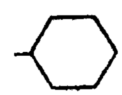 | 50,3 % | 5,00 (t)<br>2,53 (bs) | Öl |

Fortsetzung:

| | $R^1$ | $R^2$ | $R^3$ | Ausbeute | $^1H$-NMR-$CDCl_3$): /ppm | physik. Daten |
|---|---|---|---|---|---|---|
| 166 | 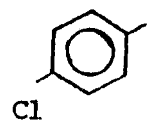 | $CH_3$ | 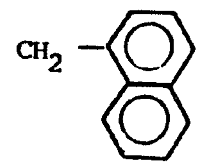 | 48 % | 4,46 (bs)<br>1,56 (s) | Öl |
| 167 | $CH_3O-\overset{O}{\overset{\|}{C}}-$ | H | 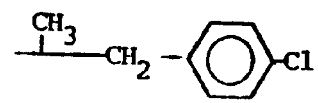 | 40 % | 4,53 (bt)<br>3,76 (s) | Öl |
| 168 | $C_2H_5O-\overset{O}{\overset{\|}{C}}-$ | H | 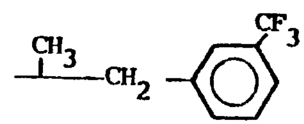 | 56 % | 4,26 (2xq)<br>1,3 (t)<br>1,0 (d) | Öl |
| 169 | $H_2N-\overset{O}{\overset{\|}{C}}-$ | H | 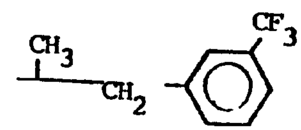 | 45 % | 4,46 (2xt)<br>1,30 (m) | Öl |
| 170 | $CH_3O-\overset{O}{\overset{\|}{C}}-$ | $CH_3$ | 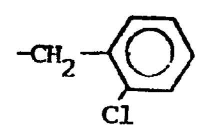 | 35 % | 4,13 (s)<br>3,43 (s)<br>1,53 (s) | Öl |

Fortsetzung:

| | $R^1$ | $R^2$ | $R^3$ | Ausbeute | $^1H$-NMR-CCD($Cl_3$): /ppm | physik. Daten |
|---|---|---|---|---|---|---|
| 171 | 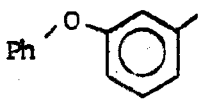 | H | 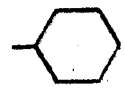 | 67 % | 4,93 (t) | Öl |
| 172 | 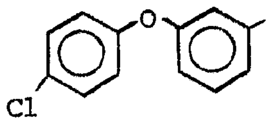 | H | 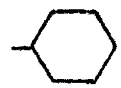 | 36 % | 4,96 (t) | Öl |
| 173 | 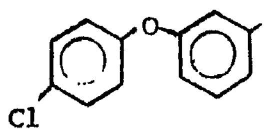 | H | 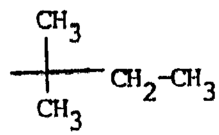 | 45 % | 4,86 (t)<br>1,06 (s) | Öl |
| 174 | 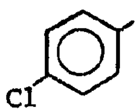 | H | 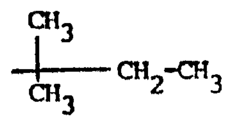 | 63 % | 4,90 (t)<br>1,06 (s) | Öl |
| 175 | 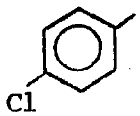 | H | 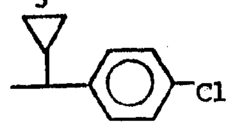 | 37 % | 4,96 (t) | Öl |
| 176 | 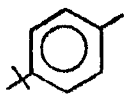 | H | 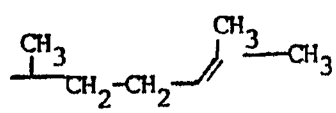 | 25 % | 4,96 (t)<br>1,30 (s)<br>1,20 (s) | Öl |

Fortsetzung:

| | $R^1$ | $R^2$ | $R^3$ | Ausbeute | $^1H$-NMR-CCD($Cl_3$): /ppm | physik. Daten |
|---|---|---|---|---|---|---|
| 177 | 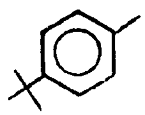 | H | 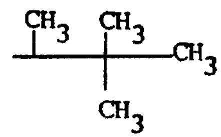 | 54 % | 4,90 (t)<br>1,33 (s)<br>0,93 (s) | öl |
| 178 | 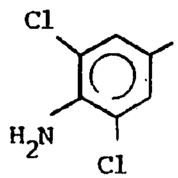 | H | $-CH_2-CH_2-CH_2-CH_3$ | 35 % | 4,86 (t)<br>0,91 (bt) | öl |
| 179 | 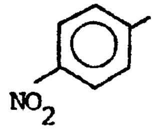 | H | 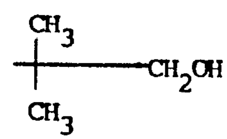 | 38 % | 85°C | |
| 180 | 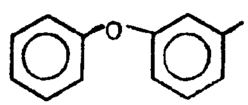 | H | 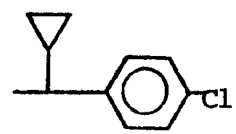 | 25 % | 4,96 (t)<br>0,33 (m) | öl |
| 181 | $HO-CH_2-$ | H | 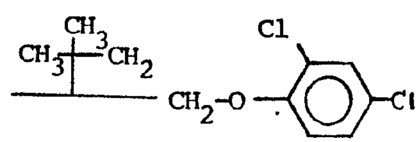 | 83 % | 1,03 (s) | öl |

Fortsetzung:

| | $R^1$ | $R^2$ | $R^3$ | Ausbeute | $^1H$-NMR-CCD$(Cl_3)$: /ppm | physik. Daten |
|---|---|---|---|---|---|---|
| 182 | 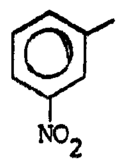 | H | 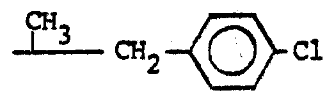 | 65 % | 5,01 (t)<br>1,01 (bd) | Öl |
| 183 | $CH_3O-\overset{O}{\overset{\|}{C}}$ | H | 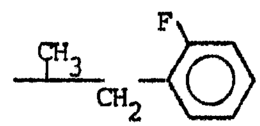 | 65 % | 4,43 (t)<br>3,68 (s) | Öl |
| 184 | 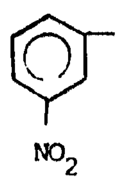 | H | 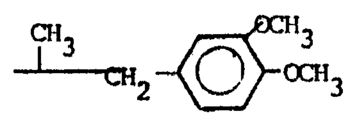 | 63 % | 5,03 (t)<br>3,80 (s)<br>1,03 (bd) | Öl |
| 185 | $CH_3O-\overset{O}{\overset{\|}{C}}-$ | $CH_3$ | 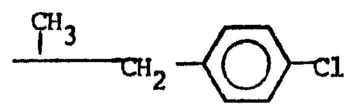 | 80 % | 3,66 (s)<br>1,46 (s) | Öl |
| 186 | 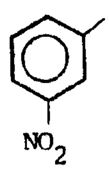 | H | 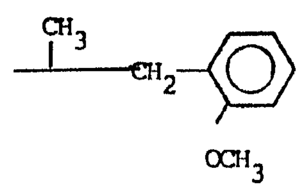 | 67 % | 5,00 (t)<br>3,73 (s) | Öl |

Fortsetzung:

| | $R^1$ | $R^2$ | $R^3$ | Ausbeute | $^1$H-NMR-CCD($Cl_3$): /ppm | physik. Daten |
|---|---|---|---|---|---|---|
| 187 | 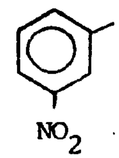 | H | 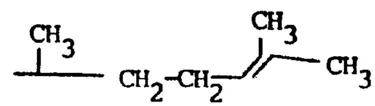 | 41 % | 3,83 (t)<br>1,13 (s) | Öl |
| 188 | 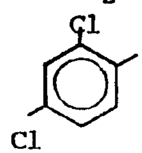 | $CONH_2$ | 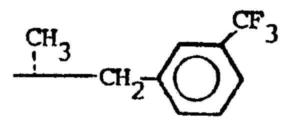 | 58 % | 5,03 (m)<br>1,03 (bd) | Öl |
| 189 | 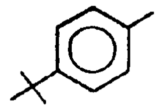 | H | 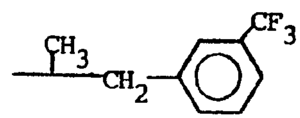 | 63 % | 5,325 (t)<br>1,33 (s) | Öl |
| 190 | 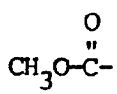 | $CH_3$ | 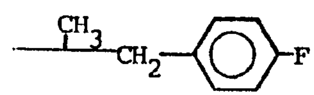 | 67 % | 3,78 (s)<br>1,53 (s)<br>0,95 (bd) | Öl |
| 191 | 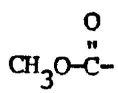 | H | 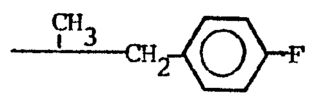 | 68 % | 4,46 (t)<br>4,37 (s)<br>2,26 (s) | Öl |

Fortsetzung:

| | $R^1$ | $R^2$ | $R^3$ | Ausbeute | $^1H$-NMR-CCD$(Cl_3)$: /ppm | physik. Daten |
|---|---|---|---|---|---|---|
| 192 | 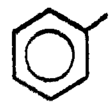 | 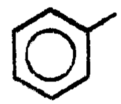 | 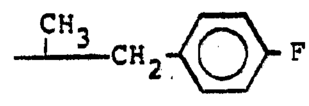 | 10 % | 7,2 (m)<br>1,01 (bd) | Öl |
| 193 | $H_2N-\overset{O}{\overset{\parallel}{C}}-$ | H | 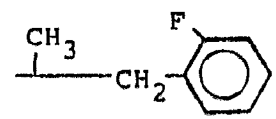 | 75 % | 4,45 (2xt)<br>1,15 (bd)<br>1,00 (d) | 48°C |
| 194 | $H_2N-\overset{O}{\overset{\parallel}{C}}-$ | H | 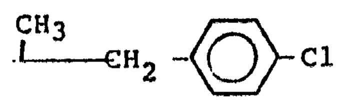 | 93 % | 4,48 (2xt)<br>1,07 (bd)<br>1,00 (d) | 83°C |
| 195 | 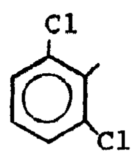 | H | 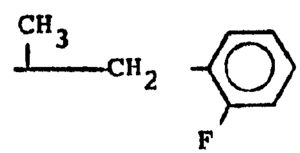 | 45 % | 5,80 (t)<br>1,05 (d) | Öl |

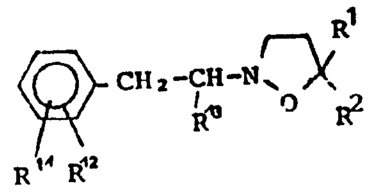

| Nr. | $R^{11}$ | $R^{12}$ | $R^{13}$ | $R^2$ | $R^1$ | 1 H-NMR CDCl/ppm |
|---|---|---|---|---|---|---|
| 196 | 4-Cl | H | $CH_3$ | 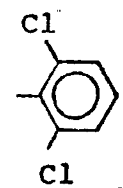 | H | 7,35-7,1 m; 5,76 t; 355-2,8 m, 1,06 d |
| 197 | 4-Cl | H | $CH_3$ | 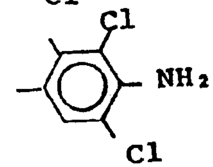 | H | 7,3-7,1 m; 4,8 t; 3,4-2,05 m; 1,03 d |
| 198 | 4-Cl | H | $CH_3$ | 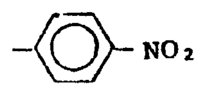 | H | 8,3-7,0 m; 5,06 t; 3,4-2,1 m; 1,03 d |
| 199 | 2-Cl | 6-Cl | $CH_3$ | 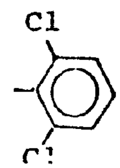 | H | 2,3-2,05 m; 5,75 t; 3,5-2,3 m; 1,1 d |

| Nr. | $R^{11}$ | $R^{12}$ | $R^{13}$ | $R^2$ | $R^1$ | 1 H-NMR CDCl/ppm |
|---|---|---|---|---|---|---|
| 200 | 3-Cl | 4-Cl | $CH_3$ | 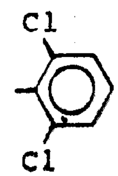 | H | 7,3-7,1 m; 5,75 t; 3,5-2,5 m; 1,03 d |
| 201 | 2-F | 5-Cl | $CH_3$ | 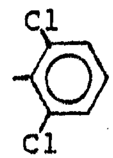 | H | 7,4-7,2 m; 5,75 t; 3,5-2,4 m; 1,88 d |
| 202 | 2-F | 6-Cl | $CH_3$ | 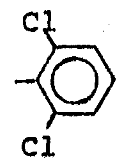 | H | 7,4-7,0 m; 5,83dxd; 3,5-2,4 m; 1,06 d |
| 203 | 2-F | 6-Cl | $CH_3$ | 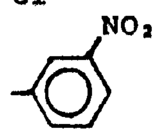 | H | 8,3-7,0 m; 5,16 t; 3,5-2,0 m; 1,10 d |
| 204 | 2-F | H | $CH_3$ | 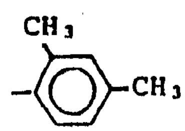 | H | 7,3-7,0 m; 5,2 t; 3,2-2,05 m; 2,3s; 1,12d |
| 205 | 2-F | H | $CH_3$ | 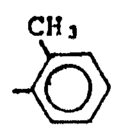 | H | 7,3-7,0 m; 5,3 t; 3,3-2,05 m; 3,36s, 3,16s, 1,1 d |

| Nr. | $R^{11}$ | $R^{12}$ | $R^{13}$ | $R^2$ | $R^1$ | 1 H-NMR CDCl/ppm |
|---|---|---|---|---|---|---|
| 206 | 2-F | H | $CH_3$ | 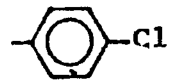 | $CH_3$ | 7,4-7,0 m; 3,5-2,4 m; 1,55s, 1,05 d |
| 207 | 2-F | H | $CH_3$ | 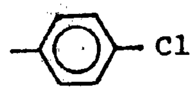 | H | 7,4-7,0 m; 5,02 t, 3,4-2,05 m; 1,15 d 1,09 d |
| 208 | 2-F | H | $CH_3$ | 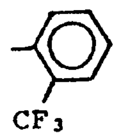 | H | 7,4-7,0 m; 5,44 t; 3,30-2,5 m; 1,12 d |
| 209 | 2-F | H | $CH_3$ | 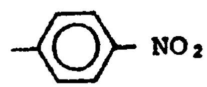 | H | 8,3-7,0 m; 5,16 t; 3,4-2,0 m; 1,10 d |
| 210 | 2-F | H | $CH_3$ | 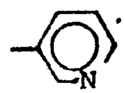 | H | |
| 211 | 2-Cl | 6-Cl | $CH_3$ | 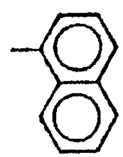 | H | |

| Nr. | $R^{11}$ | $R^{12}$ | $R^{13}$ | $R^2$ | $R^1$ | 1 H-NMR CDCl/ppm |
|---|---|---|---|---|---|---|
| 212 | 2-Cl | 6-Cl | $CH_3$ | 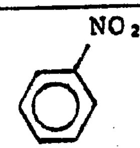 | H | |
| 213 | 3-Cl | 4-Cl | H | 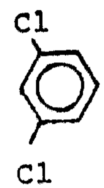 | H | |
| 214 | 3-Cl | 4-Cl | H | 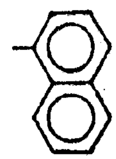 | H | |
| 215 | 3-Cl | 4-Cl | $CH_3$ | 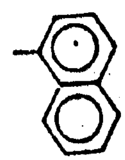 | H | |
| 216 | 2-Cl | 6-Cl | H | 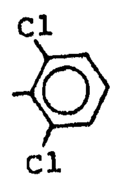 | H | |

| Nr. | $R^{11}$ | $R^{12}$ | $R^{13}$ | $R^2$ | $R^1$ | 1 H-NMR CDCl/ppm |
|---|---|---|---|---|---|---|
| 217 | 2-F | H | $-C_2H_5$ | 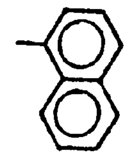 | H | |
| 218 | 2-F | H | $-C_2H_5$ | 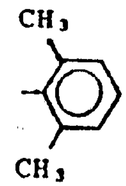 | H | |
| 219 | 2-F | H | $-C_2H_5$ | 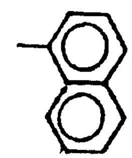 | H | |
| 220 | 2-F | H | $-C_2H_5$ | 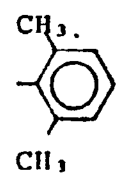 | H | |
| 221 | 3-Cl | H | $CH_3$ | 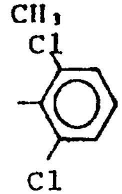 | H | |
| 222 | 2-Cl | H | $-C_2H_5$ | 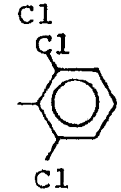 | H | |

| Nr. | $R^{11}$ | $R^{12}$ | $R^{13}$ | $R^2$ | $R^1$ | 1 H-NMR CDCl/ppm |
|---|---|---|---|---|---|---|
| 223 | 2-Cl | H | $-C_2H_5$ | 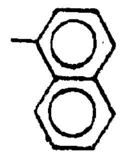 | H | |
| 224 | 2-Cl | H | $-C_2H_5$ | 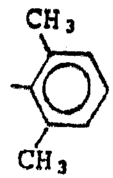 | H | |
| 225 | 2-F | H | $CH_3$ | 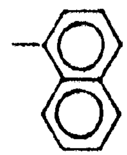 | H | 8,0-7,0 m; 5,8 m; 3,5-2,3 m; 1,1 d |
| 226 | 2-F | H | $CH_3$ | 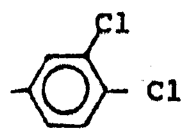 | H | 7,5-7,0 m; 5,0 t; 3,3-2,7 m; 2,1 t; 1,1 d |
| 227 | 2-F | H | $CH_3$ | 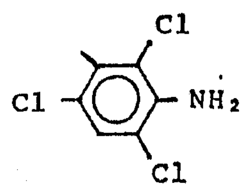 | H | 7,1-6,8 m; 5,6 t; 4,5s; 3,4-2,9 m; 2,5 m; 1,0 d |

| Nr. | $R^{11}$ | $R^{12}$ | $R^{13}$ | $R^2$ | $R^1$ | 1 H-NMR CDCl/ppm |
|---|---|---|---|---|---|---|
| 228 | 4-Cl | H | $CH_3$ | 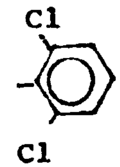 | H | 7,3-7,0 m; 5,8-5,7 m; 3,4-3,0 m; 2,7-2,5 m; 1,0 d |
| 229 | 4-$OCH_3$ | H | $CH_3$ | 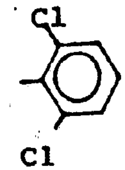 | H | 7,3-6,8 m; 5,8 t; 3,7s; 3,4-3,0 m; 2,7-2,4 m; 1,0 d |
| 230 | 2-F | H | $CH_3$ | 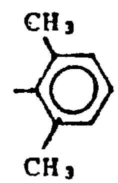 | H | 7,3-6,9 m; 5,4 t; 3,5-3,0 m; 2,5s; 1,0 d |
| 231 | 2-Br | H | $CH_3$ | 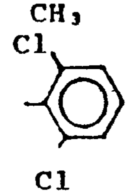 | H | 7,5-7,0 m; 5,9-5,6 m; 3,6-3,2 m; 2,8-2,5 m; 1,1 d |
| 232 | 2-$CH_3$ | H | $CH_3$ | 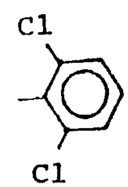 | H | 7,3-7,0 m; 5,8 t; 3,5-3,1 m; 2,7-2,5 m; 2,4s; 1,1 d |

| Nr. | $R^{11}$ | $R^{12}$ | $R^{13}$ | $R^2$ | $R^1$ | 1 H-NMR CDCl/ppm |
|---|---|---|---|---|---|---|
| 233 | 2-F | H | $CH_3$ | 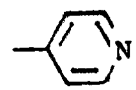 | H | 8,6 d; 7,4-7,0 m; 5,1 m; 3,4-2,6 m; 1,1 d |
| 234 | 2-F | H | $CH_3$ | 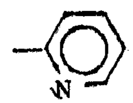 | H | 8,6 m; 7,7-7,0 m; 5,2 m; 3,4-2,6 m; 1,1 d |
| 235 | 2-F | H | $CH_3$ | 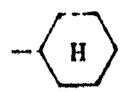 | H | 7,2-6,8 m; 3,6 m; 3,3-1,2 m; 1,0 d |
| 236 | 2-F | H | $CH_3$ | 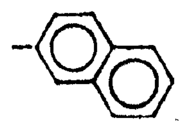 | H | 7,8-7,0 m; 5,3 m; 3,3-2,6 m; 1,1 d |
| 237 | 2-F | H | $CH_3$ | 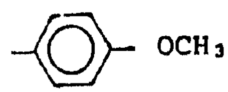 | H | 7,4-6,9 m; 5,0 m; 3,8s; 3,3-2,6 m; 1,1 d |

| Nr. | $R^{11}$ | $R^{12}$ | $R^{13}$ | $R^2$ | $R^1$ | 1 H-NMR CDCl/ppm |
|---|---|---|---|---|---|---|
| 238 | H | H | H | 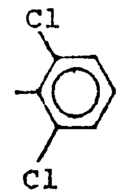 | H | 7,3-7,1 m; 5,8 m; 3,5-2,5 m |
| 239 | H | H | $CH_3$ | 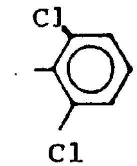 | H | 7,3-7,1 m; 5,8 t; 3,4-2,5 m; 1,1 d |
| 240 | H | H | $CH_3$ | 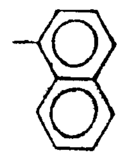 | H | 8,0-7,2 m; 5,8 m; 3,4-2,6 m; 1,1 d |
| 241 | H | H | $CH_3$ | 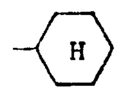 | H | 7,3-7,1 m; 3,8-3,6 m; 3,4-1,2 m; 1,0 d |

| Nr. | $R^{11}$ | $R^{12}$ | $R^{13}$ | $R^2$ | $R^1$ | 1 H-NMR CDCl/ppm |
|---|---|---|---|---|---|---|
| 242 | 2-$CH_3$ | H | $CH_3$ | 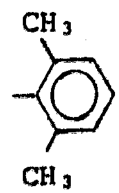 | H | 7,2-7,0 m; 5,5 t; 3,4-2,6 m; 2,4s; 1,1 d |
| 243 | 2-$CH_3$ | H | $CH_3$ | 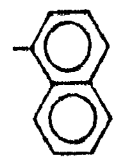 | H | 8,0-7,1 m; 5,8 m; 3,3-2,6 m; 2,4s; 1,1 d |
| 244 | 2-$CH_3$ | H | $CH_3$ | 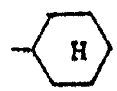 | H | 7,1 m; 3,8 m; 3,3-1,5 m; 2,4s; 1,0 d |

Herstellung der neuen Hydroxylamine der Formel V.

Beispiel a

1001 g (10 mol) Pinakolon und 822 g (11,8 mol) Hydroxylamin HCl werden mit 985 g (12 mol) Natriumacetat in 5 l Ethanol über 12 h zum Rückfluß erhitzt. Nach Abfiltrieren, Einengen am Rotationsverdampfer wird mit Ether ausgeschüttelt. Aus der Etherphase erhält man 954 g Pinakolon-Oxim vom Schmelzpunkt: 69°C (82 % Ausbeute).

115,16 g Pinakolon-Oxim (1 mol) werden in 500 ml Methanol gelöst und gegen Bromkresolgrün-Indikator mit konz. HCl auf pH 3 gebracht. 62,8 g $NaBH_3CN$ (1 mol) werden in kleinen Portionen unter Kühlung des Ansatzes mit Eis zugegeben. Dabei wird durch tropfenweise Zugabe von konz. HCl der pH-Wert bei 3 gehalten. Nach beendeter Zugabe rührt man noch 3 Stunden bei Raumtemperatur.

Durch vorsichtiges Zutropfen von konz. HCl wird der Ansatz auf pH-1 gebracht um restliches Borhydrid zu zersetzen. Man engt am Rotavapor ein (mitsamt Niederschlag) und extrahiert den Rückstand dreimal mit je 500 ml Chloroform. Nach dem Trocknen über $Na_2SO_4$ engt man das Chloroform ein. Der Rückstand kristallisiert aus.

Ausbeute: 124 g (81 %) Schmelzpunkt: 115°C (HCl-Salz)
51°C (Base)

Analog erhält man

| | Verbindung | Beispiel | Ausbeute | physik. Daten<br>$^1H$-NMR 60 MHZ<br>$CDCl_3$ |
|---|---|---|---|---|
| a | 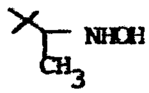 | x HCl | 81 % | Schmp.: 115°C [HCl]<br>51°C Base |
| b | 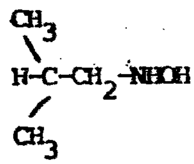 | x HCL | 85 % | 3,16 [2 x d]<br>1,10 [2 x d] |
| c | 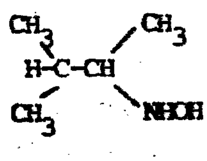 | x HCl | 80 % | 1,36 [d]<br>1,01 [2 x d] |
| d | 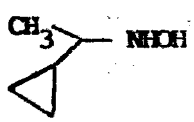 | x HCl | 80 % | 1,40 [d]<br>0,66 [mult] |
| e | 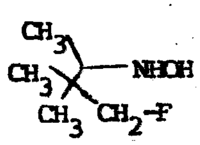 | x HCL | 85 % | 4,66 [s]<br>3,93 [s]<br>1,18 [d]<br>1,10 [d] |
| f | 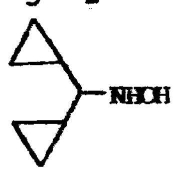 | x HCl | 60 % | Schmp.: 75°C |

| Verbindung | | Beispiel | Ausbeute | |
|---|---|---|---|---|
| j | 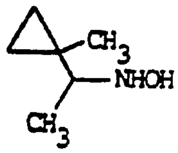 | x HCl | 40 % | 1,43 [ ]<br>1,13 [ ] |
| h | 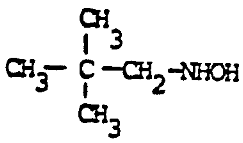 | x HCl | 80 % | 3,13 [s]<br>1,10 [s] |
| i | 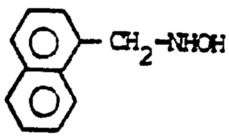 | x HCl | 76 % | Schmp.: 205°C |
| k | 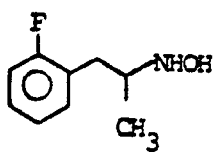 | x HCl | 25 % | Schmp.: 82°C |
| l | 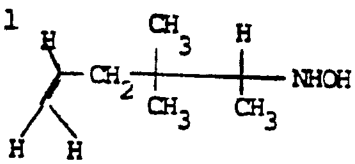 | x HCl | 59 % | 4,8-5,6 [m]<br>1,4 [illegible]<br>1,13 [s]<br>1,00 [s] |

| | Verbindung | Beispiel | Ausbeute |
| --- | --- | --- | --- |
| m | 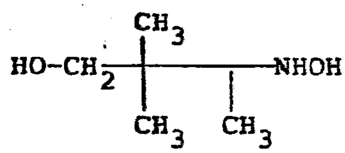 | x HCl | 62 % 3,3-3,8 [m]<br>1,40 [ ]<br>0,93 [s]<br>0,80 [s] |
| n | 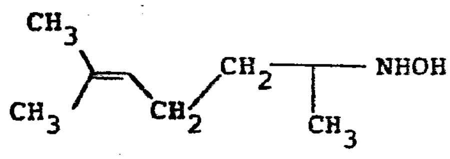 | x HCl | 98 % 3,5 [m]<br>1,01-1,8 [m] |
| o | 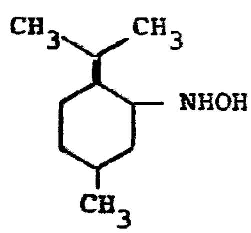 | HCl | 90 % 0,8-2,00 [m] |
| p | 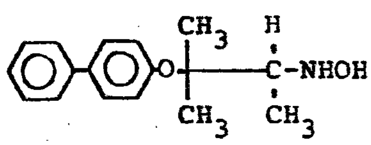 | HCl | 71 % 124 °C |
| q | 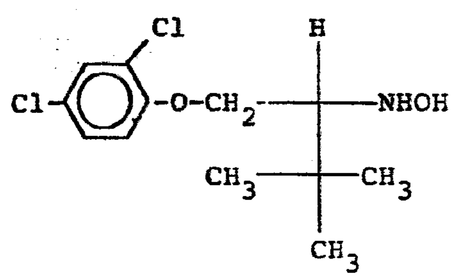 | x HCl | 86 % 139°C |

| Verbindung | Beispiel | Ausbeute | |
|---|---|---|---|
| 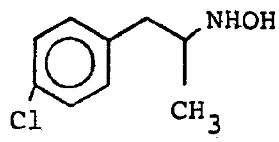 | x HCl | 75 % | Schmp. 135°C |
| 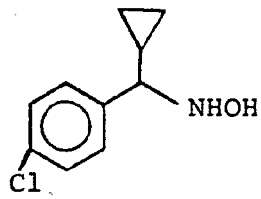 | x HCl | 78 % | 7,33 (m)<br>3,67 (bt)<br>6,70 (m) |
| 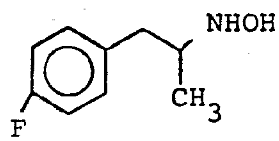 | x HCl | 85 % | 6,96 (d)<br>6,76 (d)<br>1,30 (d) |

Beispiel b

Herstellung der neuen Alkene der Formel VII
4-Amino-3,5-dichlor-styrol

4,75 g 4-Amino-3,5-dichlor-benzaldehyd (0,025 mol) werden in 50 ml Dioxan mit 13,4 g Methyl-triphenylphosphoniumbromid (0,0375 mol), 5,2 g Kaliumcarbonat unter Zusatz von 0,75 ml Wasser 3 Stunden zum Rückfluß erhitzt.

Nach dem Abkühlen saugt man vom Niederschlag ab, engt im Vakuum ein und verreibt den Rückstand mit viel Cyclohexan. Die Cyclohexanphase wird eingeengt und der Rückstand an einer kurzen Säule über Kieselgel chromatographiert. Man erhält 4,5 g (95 %) 4-Amino-3,5-dichlorstyrol vom Schmp.: 63°C.

Patentansprüche

1. Biologisch aktive Mittel, gekennzeichnet durch einen Gehalt an substituierten Isoxazolidinen der Formel I

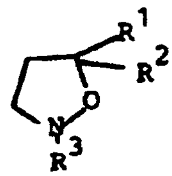

I

in welcher

$R^1$ für gegebenenfalls substituiertes Alkyl, Cycloalkyl, Aralkyl, Aryl, Heteroaryl oder Reste der Formel $-COR^2$ steht

wobei

$R^4$ für gegebenenfalls substituiertes Alkyl, Aryl, Alkoxy, Alkylthio, Aroxy, Arylthio, Amino, Alkylamino, Dialkylamino, Arylamino, Arylalkylamino, Diarylamino, Cycloalkylamino, über Stickstoff gebundene gegebenenfalls substituierte heterocyclische Reste steht,

$R^2$ für Wasserstoff sowie für die bei $R^1$ genannten Reste steht, wobei $R^1$ und $R^2$ gleich oder verschieden sein können,

$R^3$ für gegebenenfalls substituiertes Alkyl, Cycloalkyl, Alkenyl, Alkinyl, Aryl sowie für gesättigte heterocyclische Reste die gegebenenfalls substituiert sind, steht sowie deren Enantiomere und Diasteromere und Salze mit anorganischen und organischen Säuren.

2. Mittel gemäß Anspruch 1 gekennzeichnet durch einen Gehalt an Verbindungen der Formel I

in welcher

$R^1$ für $C_{1-5}$-Alkyl, $C_{3-6}$-Cycloalkyl, Phenyl-$C_{1-4}$-alkyl, Phenyl, Naphthyl, Pyridyl, steht, die ein- oder mehrfach durch Nitro, OH, CN, Halogen, $C_{1-4}$-Alkyl, $C_{3-6}$-Cycloalkyl, $C_{1-4}$-Alkoxy, $C_{1-4}$-Alkylthio, $C_{1-2}$-Alkylendioxy, $C_{1-4}$-Halogenalkyl, $C_{1-4}$-Alkoxycarbonyl, Carboxyl (COOH), Amine, $C_{1-4}$-Alkylamino, Di-$C_{1-4}$-alkylamino, Arylamino, Aryl-$C_{1-4}$-alkylamino, Aminocarbonyl-$C_{1-4}$-alkyl (-NHCO$C_{1-4}$-alkyl), $C_{1-4}$-Alkylsulfonylamino (-$NHSO_2C_{1-4}$-alkyl), $C_{1-4}$-Halogenalkoxy, $C_{1-4}$-Halogenalkylthio, $C_{1-4}$-Alkylendioxy, $C_{1-4}$-Halogenalkylendioxy, Aryl wie Phenyl, Aryloxy wie Phenyl substituiert sein können, sowie für einen Rest der Formel $-COR^4$ steht, wobei $R^4$ für $C_{1-4}$-Alkyl, Phenyl, $C_{1-4}$-Alkylthio, Phenoxy, Phenylthio, Amino, $C_{1-4}$-Alkylamino, Di-$C_{1-4}$-alkylamino, Phenylamino, Naphthylamino, Phenyl-$C_{1-4}$-alkylamino, Diphenylamino, $C_{3-6}$-Cycloalkylamino, Morpholino, Piperidino, Pyridino, Imidazolino, Piperzino, Triazolino steht, wobei diese Reste durch einen oder mehrere der oben aufgeführten Substituenten substituiert sein können,

$R^2$ für Wasserstoff steht oder für einen der unter $R^1$ angegebenen Reste steht, wobei $R^1$ und $R^2$ gleich oder verschieden sein können,

$R^3$ für $C_{1-10}$-Alkyl, $C_{3-6}$-Cycloalkyl, $C_{2-4}$-Alkenyl, $C_{2-6}$-Alkinyl, Phenyl, Naphthyl, steht, für $C_{1-6}$-Alkyl steht das durch Halogen, OH, CN, Amino, $C_{1-4}$-Alkylamino, $C_{1-4}$-Dialkylamino, $C_{1-4}$-Alkoxy, $C_{3-6}$-Cycloalkyl, gegebenenfalls substituiertes Phenyl, Naphthyl, Phenoxy, Phenylthio, Oxycarbonyl-$C_{1-4}$-alkyl, CCO-$C_{1-4}$-Alkyl, substituiert ist, für Phenyl das durch Halogen, $NO_2$, CN, OH, Amino, $C_{1-4}$-Alkylamino, Di-$C_{1-4}$-alkylamino, Carbonyl-($C_{1-4}$-alkoxy), (-COO$C_{1-4}$-Alkyl) substituiert ist, für gegebenenfalls substituiertes Phenoxy, Naphthoxy, Biphenylyloxy steht.

3. Mittel gemäß Anspruch 1 gekennzeichnet durch einen Gehalt an

in welcher

$R^1$ für gegebenenfalls substituiertes Phenyl, Pyridyl, $C_{1-5}$-Alkyl, Naphthyl, sowie für einen Rest der Formel $-COR^4$ steht, wobei $R^4$ für $C_{1-4}$-Alkoxy insbesondere Methoxy oder Ethoxy steht.

Ferner steht $R^1$ für ein oder mehrfach gleich oder verschieden substituiertes Phenyl. Als Substituenten seien genannt Halogen wie Fluor, Chlor, Brom, $C_{1-4}$-Alkyl insbesondere Methyl,

Propyl, Methoxy, Nitro, Trifluormethyl, Trifluormethoxy, CN, $-COOCH_3$, COOH, Amino, $-NHCOCH_3$, Methylendioxy, Phenoxy das gegebenenfalls halogensubstituiert ist.

Ferner steht $R^1$ für $C_{1-5}$-Alkyl das ein- oder mehrfach substituiert ist durch Halogen, Cyclopropyl, Cyclohexyl, Hydroxy, $C_{1-4}$-Alkoxy insbesondere Methoxy, Phenyl das gegebenenfalls durch Halogen, Methoxy substituiert ist.

Ferner steht $R^1$ für Pyridyl das durch Methyl oder Halogen, insbesondere Chlor substituiert ist.

$R^2$ für Wasserstoff, Phenyl, $C_{1-4}$-Alkyl insbesondere Methyl, Propyl, Chlorphenyl, sowie für den Rest der Formel $-COR^4$ steht wobei $R^4$ für $C_{1-4}$-Alkoxy, insbesondere Methoxy oder Ethoxy, Di-$C_{1-4}$-alkylamino insbesondere Diethylamin steht

$R^3$ für $C_{1-6}$-Alkyl insbesondere Methyl, Ethyl, i-Propyl, t-Butyl, i-Pentyl, Pinalkolyl, gegebenenfalls substituiert durch Cyclopropyl, Halogen, insbesondere Fluor, OH, Phenyl das gegebenenfalls durch Fluor, Trifluormethyl, substituiert ist, sowie für gegebenenfalls substituiertes Naphthyl, Phenyl, Halogenphenoxy, Naphthoxy, Biphenylyloxy steht.

4. Schädlingsbekämpfungsmittel gekennzeichnet durch einen Gehalt an Verbindungen der Formel I

in welcher

$R^3$ für gegebenenfalls substituiertes verzweigtes Alkyl steht. Als Substituenten kommen dabei Halogen, insbesondere Chlor oder Fluor, Cyclopropyl, Cyclohexyl, Hydroxy, $C_{1-4}$-Alkoxy insbesondere Methoxy, Phenyl das gegebenenfalls durch Halogen oder Methoxy substituiert ist,

$R^1$ und $R^2$ die in Ansprüchen 1-3 genannten Bedeutungen besitzen.

5. Futterzusatzmittel gekennzeichnet durch einen Gehalt an Verbindungen der Formel I

in welcher

$R^1$ für gegebenenfalls substituiertes Aryl steht, wobei die beiden vorzugsweise Definitionen genannten Reste auch hier besonders bevorzugt sind,

$R^2$ für Wasserstoff steht und

$R^3$ die in Ansprüchen 1-3 genannten Bedeutungen besitzen.

6. Verbindungen der Formel I

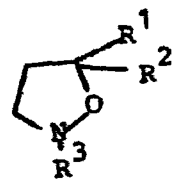

I

in welcher

$R^1$ und $R^2$ die in Ansprüchen 1-3 angegebenen Bedeutungen besitzen und

$R^3$ für Alkyl mit mehr als 3 C-Atomen sowie für substituiertes Alkyl ferner für gegebenenfalls substituiertes Cycloalkyl, Alkenyl, Alkinyl, Aryl sowie für gesättigte oder ungesättigte heterocyclische Reste die gegebenenfalls substituiert sind steht,

sowie ihre Salze und Enantiomeren und Diasteromeren.

7. Verfahren zur Herstellung von Verbindungen der Formel I

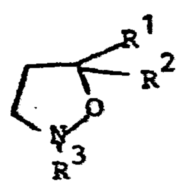

I

in welcher

$R^3$ für Alkyl mit mehr als 3 C-Atomen sowie für substituiertes Alkyl ferner für gegebenenfalls substituiertes Cycloalkyl, Alkenyl, Alkinyl, Aryl sowie für gesättigte oder ungesättigte heterocyclische Reste die gegebenenfalls substituiert sind steht,

dadurch gekennzeichnet, daß man Hydroxylamine der Formel II

$R^3$-NH-OH    II

in welcher

$R^3$ die oben angegebene Bedeutung hat,

mit Formaldehyd und Alkenen der Formel III

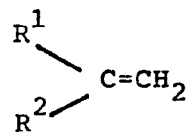

III

in welcher

$R^1$ und $R^2$ die oben angegebene Bedeutung haben,

umsetzt.

8. Hydroxylamine der Formel V

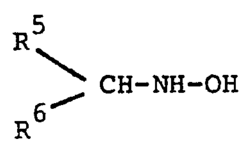

V

in welcher

$R^5$ für Alkyl, Cycloalkyl, Alkenyl, Alkinyl, die gegebenenfalls durch Halogen, Aryloxy, Alkoxy, Halogenalkyl, Halogenalkoxy substi-

0162383

tuiert sind, ferner für durch Halogen, Alkoxy, Alkyl, Halogenalkyl, Halogenalkoxy substituiertes Aryl oder Aralkyl steht,

$R^6$ unabhängig von $R^5$ die dort angegebenen Bedeutungen hat,

sowie Salze mit anorganischen und organischen Säuren dieser Verbindungen.

9. Verfahren zur Herstellung der Hydroxylamine der Formel V dadurch gekennzeichnet, daß man Oxime der Formel VI

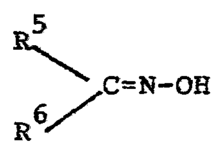

VI

in denen die Reste

$R^5$ und $R^6$ die oben angegebene Bedeutung haben

mit $NaBH_3CN$ in schwach saurer alkoholischer Lösung reduziert.

10. Alkene der Formel VII

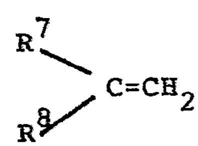

VII

in welcher

$R^7$ für Wasserstoff steht,

$R^8$ für Reste der Formeln steht,

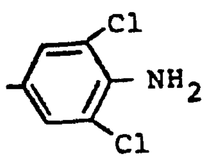 , 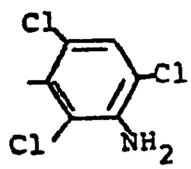 , 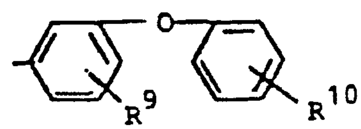

wobei

$R^9$, $R^{10}$ unabhängig voneinander für Wasserstoff oder Halogen, Trifluormethyl stehen, wobei einer der Reste $R^9$ oder $R^{10}$ eine andere Bedeutung als Wasserstoff haben muß.

11. Verfahren zur Herstellung der Alkene der Formel VII dadurch gekennzeichnet, daß man

a) Halogenalkyl der Formel VIII

$$R^8\text{-}CH_2\text{-}Hal \qquad VIII$$

in welcher

$R^8$ die oben angegebene Bedeutung hat

mit Triphenylphosphin und anschließend in Gegenwart einer Base mit Formaldehyd umsetzt oder

b) einen Aldehyd der Formel IX

$$R^8\text{-CHO} \qquad \text{IX}$$

in welcher

$R^8$ die oben angegebene Bedeutung hat

in einer Wittig-Reaktion olefiniert.

12. Verwendung von substituierten Isoxazolidinen der Formel (I) gemäß Anspruch 1 zur Bekämpfung von Schädlingen.

13. Verfahren zur Herstellung von Schädlingsbekämpfungsmitteln, dadurch gekennzeichnet, daß man substituierte Isoxazolidine der Formel (I) gemäß Anspruch 1 mit Streckmitteln und/oder oberflächenaktiven Mitteln vermischt.

14. Verwendung von substituierten Isoxazolidinen der Formel I gemäß Anspruch 1 als Futterzusatzmittel.

15. Verfahren zur Herstellung von Futter oder Futterzusatzmitteln, dadurch gekennzeichnet, daß man substituierte Isoxazolidine der Formel I gemäß Anspruch 1 mit Futter, üblichen Futterzusatzstoffen, Streckmitteln oder Hilfsstoffen vermischt.

Europäisches Patentamt

# EUROPÄISCHER RECHERCHENBERICHT

0162383
Nummer der Anmeldung

EP 85105761.2

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | DE - A1 - 2 317 980 (TEIKOKU)<br>* Ansprüche 2,3 *<br>-- | 6 | C 07 D 261/02<br>A 01 N 43/80<br>A 23 K 1/16<br>A 23 K 1/18<br>C 07 C 83/00<br>C 07 C 87/60<br>C 07 C 85/24 |
| A | CHEMICAL ABSTRACTS, Band 79, Nr. 5, 6. August 1973, Columbus, Ohio, USA<br>LAUGHLIN, R.G. "Reversible cyclo-elimination and disproportionation reactions in aliphatic amine oxide-N,N-dimethylhydroxylamineolefin systems."<br>Seite 382, Spalte 2, Zusammenfassung-Nr. 31 132e<br>& J. Amer. Chem. Soc., 1973, 95(10), 3295-9<br>-- | 6 | |
| A | CHEMICAL ABSTRACTS, Band 97, Nr. 21, 22. November 1982, Columbus, Ohio, USA<br>DE SHONG, PHILIP; DICKEN, C. MICHAEL; STAIB, RONALD R.; FREYER, ALAN J.; WEINREB, STEVEN M. "Determination of configuration and conformation of isoxazolidines by nuclear Overhauser effect difference spectroscopy."<br>Seite 732, Spalte 2, Zusammenfassung-Nr. 181 379b<br>& J. Org. Chem. 1982, 47(23), 4397-403<br>-- | 6 | RECHERCHIERTE SACHGEBIETE (Int. Cl.4)<br>C 07 D 261/00<br>C 07 C 83/00<br>C 07 C 85/00<br>C 07 C 87/00<br>A 01 N 43/00<br>A 23 K 1/00 |
| X | CH - A - 344 073 (INVENTA)<br>* Beispiel 2 *<br>-- | 8 | |

Der vorliegende Recherchenbericht wurde für alle Patentansprüche erstellt.

| Recherchenort | Abschlußdatum der Recherche | Prüfer |
|---|---|---|
| WIEN | 19-07-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0162383
Nummer der Anmeldung

EP 85105761.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | BEILSTEINS HANDBUCH DER ORGANISCHEN CHEMIE, 4. Auflage, 1928, Band E I 1<br>SPRINGER VERLAG, Berlin<br>Seite 188<br>-- | 8 | |
| X | BEILSTEINS HANDBUCH DER ORGANISCHEN CHEMIE, 4. Auflage, 1973, Band E IV 1<br>SPRINGER VERLAG, Berlin<br>Seiten 1773, 1774<br>-- | 8 | |
| X | FIESER, FIESER: "Reagents for Organic Synthesis", 1977, Band 6<br>VERLAG JOHN WILEY, New York, Seite 538<br>-- | 9 | RECHERCHIERTE SACHGEBIETE (Int. Cl.4) |
| A | DE - B2 - 2 362 780 (BAYER)<br>* Beispiel 2 *<br>---- | 10 | |

Der vorliegende Recherchenbericht wurde für alle Patentansprüche erstellt.

| Recherchenort | Abschlußdatum der Recherche | Prüfer |
|---|---|---|
| WIEN | 19-07-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82